# EUROPÄISCHE PATENTSCHRIFT

(11) **EP 3 585 242 B1**
(45) Veröffentlichungstag und Bekanntmachung des Hinweises auf die Patenterteilung: **27.10.2021**
(21) Anmeldenummer: 18710781.8
(22) Anmeldetag: 22.02.2018
(51) Int. Cl.: A61B 3/00, A61F 9/009

(54) **PATIENTENINTERFACE UND VERFAHREN ZUM SICHERSTELLEN DER STERILITÄT DES PATIENTENINTERFACES**
PATIENT INTERFACE AND METHOD FOR ENSURING THE STERILITY OF SAID PATIENT INTERFACE
INTERFACE PATIENT ET PROCÉDÉ POUR GARANTIR LA STÉRILITÉ DE L'INTERFACE PATIENT

(30) Priorität: 27.02.2017 DE 102017103999
(43) Veröffentlichungstag der Anmeldung: 01.01.2020
(73) Patentinhaber: Carl Zeiss Meditec AG, 07745 Jena (DE)
(72) Erfinder: RILL, Michael Stefan, 07751 Jena (DE); DAMM, Tobias, 81545 München (DE)
(74) Vertreter: Patentanwälte Geyer, Fehners & Partner mbB
(86) Internationale Anmeldenummer: PCT/EP2018/054426
(87) Internationale Veröffentlichungsnummer: WO 2018/154009

(56) Entgegenhaltungen:
- WO-A1-2012/031277
- WO-A1-2013/167274
- DE-A1-102015 002 726
- JP-A- 2013 248 303
- US-A1- 2007 173 791
- US-A1- 2009 131 921

## Beschreibung

Die Erfindung betrifft ein Verfahren zum Sicherstellen der Sterilität eines Patienteninterfaces, das an einer ophthalmologischen Vorrichtung eingesetzt wird, wobei das Patienteninterface einerseits zur Anlage an einem Auge eines Patienten und andererseits zur Befestigung an der ophthalmologischen Vorrichtung ausgebildet ist. Ferner betrifft die Erfindung ein Patienteninterface zum Verbinden eines Auges eines Patienten mit einer ophthalmologischen Vorrichtung.

In den letzten Jahren wurden zahlreiche ophthalmologische Vorrichtungen in den Markt eingeführt, mit dem Ziel, menschliches Augengewebe automatisiert zu schneiden, abzutragen oder zu koagulieren. Dabei kommen je nach Anwendung verschiedene Laser-Gewebe-Wechselwirkungen zum Einsatz, wie zum Beispiel Photokoagulation, Photoablation, plasmagestützte Ablation und Photodisruption. Um nur bestimmte Gewebestrukturen des Auges zu behandeln und das Patientenauge dabei nicht zu verletzen, ist es bei vielen ophthalmologischen Vorrichtungen wichtig, das Patientenauge mechanisch zu fixieren. Im Falle einer mechanischen Stabilisierung werden Patienteninterfaces eingesetzt, die eine mechanische und zugleich optische Schnittstelle zwischen dem Augen des Patienten und der ophthalmologischen Vorrichtung darstellen. Solche Patienteninterfaces werden üblicherweise mithilfe von Unterdruck am Auge festgesaugt und mechanisch oder wiederum mittels Unterdruck an der ophthalmologischen Vorrichtung befestigt. Das Patienteninterface verbindet so das Auge des Patienten mit einem optischen Ausgang der ophthalmologischen Vorrichtung. In dieser Position - befestigt an der ophthalmologischen Vorrichtung und am Auge des Patienten - hat das Patienteninterface sowohl die Funktion des letzten optischen Elements einer Optik der ophthalmologischen Vorrichtung als auch einer mechanischen Fixierung, die das Auge relativ zu der ophthalmologischen Vorrichtung fixiert. Dabei kann das Patienteninterface Zuleitungen aufweisen, um es an der ophthalmologischen Vorrichtung und/oder am Auge anzusaugen und/oder um Flüssigkeiten zwischen das Auge und das Patienteninterface zu bringen.

Da Patienteninterfaces im direkten Kontakt zu menschlichem Gewebe, z. B. menschlicher Kornea oder Sklera, stehen und je nach Anwendungsfall über Zugangsschnitte das Augeninnere mit Sporen oder Keimen infizieren können, müssen sie steril appliziert werden. Grundsätzlich könnte man ein Patienteninterface nach einer Verwendung desinfizieren oder erneut sterilisieren und somit mehrfach - also auch bei weiteren Patienten - verwenden. Die meisten kommerziell erhältlichen Patienteninterfaces sind jedoch nicht dafür vorgesehen und als Wegwerfartikel ausgelegt. Eine Wiederverwendung wäre unter hygienischen Gesichtspunkten äußerst bedenklich und soll verhindert werden.

Die DE 10 2015 002 726 A1 beschreibt eine ophthalmologische Lasertherapievorrichtung und ein Verfahren zur Kalibrierung. Die WO 2013/167274 A1 beschreibt einen Aufsatz für ein Kontaktglas sowie ein Herstellungsverfahren für ein Kontaktglassystem. Die US 2009/131921 A1 beschreibt bildgeführte Operationstechniken, einen Operationsapparat und ein Operationssystem und die US 2007/173791 A1 beschreibt ein System für ophthalmologische Laseroperationen.

Aufgabe der Erfindung ist es, ein Patienteninterface und ein Verfahren zum Anbringen und Lösen eines Patienteninterfaces an einer ophthalmologischen Vorrichtung bereitzustellen, so dass eine Einmalverwendung des Patienteninterfaces gesichert ist.

Die Erfindung ist in den unabhängigen Ansprüchen definiert. Die abhängigen Ansprüche betreffen bevorzugte Weiterbildungen.

Beim Verfahren zum Sicherstellen der Sterilität eines Patienteninterfaces ist ein Patienteninterface vorgesehen, das an einer ophthalmologischen Vorrichtung befestigt ist, welche zur Untersuchung und/oder Behandlung eines Auges eines Patienten ausgebildet ist. Das Patienteninterface ist zur Vakuumfixierung am Auge des Patienten ausgebildet. Um eine Wiederverwendung des Patienteninterfaces zu verhindern, wird mittels der ophthalmologischen Vorrichtung eine Strukturveränderung am Patienteninterface vorgenommen. Diese Strukturveränderung modifiziert das Patienteninterface hinsichtlich bestimmter optischer oder elektrischer oder Fluiddichtigkeitseigenschaften. Unter Fluid wird dabei sowohl ein Gas als auch eine Flüssigkeit verstanden. Patienteninterfaces, wie sie insbesondere in der Katarakt-OP eingesetzt werden, haben eine Flüssigkeitskammer zum Auge hin, die nach dem Befestigen des Patienteninterfaces am Auge mit einer Flüssigkeit gefüllt wird, welche für einen möglichst guten Übergang der Brechzahl zwischen optischen Elementen des Patienteninterfaces und der Augenhornhaut sorgt.

Als Strukturveränderungen kommen verschiedene Eingriffe am Patienteninterface in Frage, die nachfolgend noch näher erläutert werden. Die Strukturveränderungen lassen sich in zwei Klassen einteilen. Eine erste Klasse von Strukturveränderungen ist mittels der ophthalmologischen Untersuchungs- und/oder Behandlungsvorrichtung detektierbar, ändert das Patienteninterface jedoch nicht derart, dass es funktionell nicht einsetzbar wäre. Eine zweite Klasse modifiziert das Patienteninterface so, dass es aus technischen Gründen nicht mehr an der ophthalmologischen Untersuchungs- und/oder Behandlungsvorrichtung eingesetzt werden kann, beispielsweise weil es nicht mehr an der Aufnahme befestigt werden kann.

Während die zweite Klasse von Strukturveränderungen eine Wiederverwendung des Patienteninterfaces schon grundsätzlich ausschließt (es kann ja funktionell nicht mehr eingesetzt werden), wäre für die erste Klasse an Veränderungen eine Wiederverwendung theoretisch möglich. Es ist deshalb auch ein Verfahren vorgesehen, das am Patienteninterface vor der Untersuchung und/oder Behandlung eine Überprüfung durchführt. Hierbei wird überprüft, ob das Patienteninterface eine Strukturveränderung aufweist, die eine Wiederverwendung anzeigt. Eine solche Strukturveränderung modifiziert das Patienteninterface hinsichtlich bestimmter optischer oder elektrischer oder Fluiddichtigkeitseigenschaften. Wurde eine Wiederverwendung erkannt, wird der weitere Betrieb der ophthalmologischen Vorrichtung verhindert. Diese Überprüfung auf Wiederverwendung kann je nach Ausgestaltung eine Prüfung auf nur eine Strukturveränderung umfassen oder eine Überprüfung auf eine Gruppe von Strukturveränderungen.

Analog zu den geschilderten Verfahren ist eine ophthalmologische Untersuchungs- und/oder Behandlungsvorrichtung vorgesehen, die eine Aufnahme zum Anbringen eines Patienteninterfaces aufweist. Das Patienteninterface dient zum Verbinden eines Auges eines Patienten mit der Vorrichtung. Die Vorrichtung ist ausgebildet, zur Verhinderung der Wiederverwendung des Patienteninterfaces eine Strukturveränderung am Patienteninterface vorzunehmen. Die Strukturveränderung modifiziert das Patienteninterface hinsichtlich bestimmter optischer oder elektrischer oder Fluiddichtigkeitseigenschaften.

Weiter ist ein Patienteninterface zum Verbinden eines Auges eines Patienten mit einer ophthalmologischen Untersuchungs- und/oder Behandlungsvorrichtung vorgesehen. Es ist zur Unterdruckbefestigung am Auge und/oder der Vorrichtung ausgebildet und hat mindestens eine Fluidleitung zur Befestigung an einem Anschluss der Vorrichtung. Die Fluidleitung weist einen Verbinder auf. Verbinder und/oder der Anschluss sind für eine Einwegverbindung ausgestaltet. Nach dem erstmaligen Entfernen der Fluidleitung vom Anschluss kann kein erneutes fluiddichtes Anschließen erfolgen. Beispielsweise ist der Verbinder als Einwegverbinder ausgeführt. Alternativ weist das Patienteninterface eine zur Unterdruckbefestigung benötigte Wandung auf, in der eine Soll-Bruchstelle vorgesehen ist. Dies ist so gestaltet, dass sie bei einem vorgegebenen Differenzdruck bricht. Ein solches Patienteninterface kann sehr einfach funktionell für eine Wiederverwendung blockiert werden, indem nach dem Ersteinsatz ein Unterdruck so eingestellt wird, dass der vorgegebene Differenzdruck an der Soll-Bruchstelle überschritten wird. Die Wandung hat dann ein Loch, wodurch eine erneute Unterdruckbefestigung bei einer Wiederverwendung des Patienteninterfaces fehlschlägt.

Soweit nachfolgend auf das Erzeugen einer Strukturveränderung im Patienteninterface abgestellt wird, ist davon natürlich gleichermaßen auch die Überprüfung auf Wiederverwendung betroffen, die dann vor dem Untersuchungs- und/oder Behandlungsbetrieb prüft, ob eine entsprechende Strukturveränderung am Patienteninterface vorhanden ist.

Natürlich muss bei der Überprüfung auf Wiederverwendung darauf geachtet werden, dass nicht irrtümlich eine Strukturveränderung detektiert wurde, die in der aktuellen Verwendung erzeugt wurde. Dies kann zum einen dadurch sichergestellt werden, dass die Überprüfung auf Wiederverwendung vor dem Einbringen der Strukturveränderung erfolgt. Zum anderen ist es auch möglich, zu überprüfen, ob eine ältere Strukturveränderung vorliegt, die sich von der Strukturveränderung unterscheidet, die zuvor in der aktuellen Verwendung des Patienteninterfaces eingebracht wurde. Detektierte die Überprüfung auf Wiederverwendung eine Strukturveränderung im Patienteninterface, wird optional ein Signal erzeugt, das dem Benutzer anzeigt, dass das aktuell an der ophthalmologischen Vorrichtung angebrachte Patienteninterface bereits verwendet wurde. Eine Strukturveränderung, die optisch erkennbar ist, kann beispielsweise mithilfe eines Detektionsverfahrens, z. B. softwaregestützt, erkannt werden, das die optische Qualität des Elementes, z. B. der Linse, testet. Beispielsweise kann ein OCT- oder Scheimpflug-Scan des transparenten Elements des Patienteninterfaces erfolgen. Ferner ist es möglich, ein Bild des transparenten Elements und/oder der Fassung zu erzeugen und mittels Bildvergleichs zu erkennen, ob eine Strukturveränderung vorhanden ist. Es ist auch möglich, den Laserstrahl zur Behandlung des Auges (optional bei abgeschwächter Intensität) durch das transparente Element zu führen und zu überprüfen, ob Reflexionen an dem transparenten Element die Strukturveränderungen anzeigen. Verschiedene Ausführungsformen, wie diese Unterscheidung erfolgen kann, werden nachfolgend noch beschrieben.

Es ist durch alle Ausführungsformen sichergestellt, dass das Patienteninterface nicht erneut verwendet werden kann. Dies erfolgt unabhängig von Kenntnissen und/oder Aufmerksamkeit des Verwenders, welcher das Patienteninterface an der ophthalmologischen Vorrichtung anbringt. Somit ist die Zuverlässigkeit erhöht und die Hygiene sichergestellt. Dies geschieht unabhängig von der Präsenz eines Patienten, da auf das Anbringen und Lösen des Patienteninterfaces an oder von der ophthalmologischen Vorrichtung und nicht auf das Befestigen am Auge abgestellt ist. Die Strukturveränderung geschieht ohne Mitwirkung des Patienten. Er muss zur Durchführung des Verfahrens nicht anwesend sein.

Die ophthalmologische Vorrichtung kann Augengewebe, z. B. durch Photokoagulation, Photoablation, plasmagestützte Ablation und/oder Photodisruption, behandeln. Die ophthalmologische Vorrichtung weist in Ausführungsformen eine Laserquelle zur Bereitstellung eines Laserstrahls zur Behandlung des Auges und eine Laserstrahlführeinrichtung auf, die die Lage eines Fokus des Laserstrahls im Auge verstellt. Die ophthalmologische Vorrichtung wird beispielsweise zur Behandlung der Retina, zur Kataraktoperation und/oder zur Korrektur von Fehlsichtigkeit verwendet. Die ophthalmologische Vorrichtung kann ein Operationsmikroskop umfassen, mittels welchem das Auge untersucht werden kann. Ferner kann die Vorrichtung eine OCT-Einrichtung zur Erzeugung eines OCT-Bilds des Auges eine Scheimpflugkamera oder ein Ultraschallgerät zur Behandlung und/oder Untersuchung des Auges aufweisen. Die ophthalmologische Vorrichtung weist eine Aufnahme auf, an der das Patienteninterface befestigt wird. Das Patienteninterface bildet eine Schnittstelle zwischen der ophthalmologischen Vorrichtung und dem Auge.

Die Strukturveränderung ist in einigen Ausführungsformen derart ausgestaltet, dass sie mit bloßem Auge sichtbar ist. Sie stellt dann eine Entwertungsmarkierung dar. In anderen Ausführungsformen beeinflusst die Strukturveränderung Strahlung zur Behandlung und/oder Beobachtung des Auges derart, dass eine Behandlung und/oder Beobachtung des Auges mit der ophthalmologischen Vorrichtung nur noch eingeschränkt oder gar nicht möglich ist.

In Ausführungsformen weist das Patienteninterface ein transparentes Element auf, das in einer Fassung gehalten ist und durch das im Betrieb der ophthalmologischen Vorrichtung Strahlung zur Beobachtung und/oder Behandlung des Auges läuft, und die Strukturveränderung wird im transparenten Element und/oder der Fassung erzeugt. Das transparente Element kann beispielsweise eine Linse des Patienteninterfaces sein. Die Fassung umgibt das transparente Element. Sie kann ebenfalls transparent sein; es ist jedoch auch möglich, dass die Fassung opak oder lichtundurchlässig ausgestaltet ist, da im Bereich der Fassung keine Strahlung zur Beobachtung und Behandlung des Auges geführt wird. Das transparente Element und/oder die Fassung kann/können aus Kunststoff hergestellt sein.

Bevorzugt wird die Strukturveränderung durch die Laserstrahlung der ophthalmologischen Vorrichtung erzeugt. Eine per Laser erzeugte Strukturveränderung kann eine geometrische Figur, z. B. einen Strich, einen Punkt, ein Kreuz, einen Kreis, ein Rechteck, ein Quadrat, eine Ellipse oder eine Kombination davon umfassen. Ferner kann die Figur auch flächig in das transparente Element und/oder in die Fassung eingebracht werden. Ferner kann die erzeugte Strukturveränderung auch einen Code (z. B. Strichcode) und/oder einen Schriftzug realisieren, der beispielsweise das Datum, die Uhrzeit und/oder den Namen eines Patienten angibt, bei dem das Patienteninterface bereits genutzt wurde. Dies hat den Vorteil, dass das entsprechende Patienteninterface archiviert werden kann und später zur Nachvollziehbarkeit und Protokollierung der Behandlung des Patienten herangezogen werden kann. Die Laserquelle kann eine Femtosekundenlaserquelle oder eine Laserquelle sein, die zur Behandlung des Auges bei verschiedenen Krankheiten verwendet wird und die auch das Element oder die Fassung bearbeiten kann, um die Strukturveränderung zu erzeugen. Die Laserstrahlführeinrichtung kann in Ausführungsformen mindestens einen Scanner und eine Optik zur dreidimensionalen Verstellung der Lage des Fokus umfassen. Die Laserstrahlführeinrichtung ist bevorzugt derart ausgestaltet, dass sie den Fokus des Laserstrahls nicht nur innerhalb des zu behandelnden Gewebes des Auges verstellen, sondern auch in das Patienteninterface bewegen kann, z. B. indem sie über einen entsprechend großen Fokusverstellbereich oder ein zusätzliches optisches Element (z. B. Spiegel oder Linse), das in einen Strahlengang des Laserstrahls eingeführt wird und somit eine Verschiebung des Fokus des Laserstrahls vom Auge in Strukturen des Patienteninterfaces bewirkt, verfügt. Das Erzeugen der Strukturveränderung, z. B. der genannten Entwertungsmarkierung, erfolgt dann durch Führen des Fokus des Laserstrahls in Strukturen des Patienteninterfaces. Dies kann nach Abschluss des Behandlungs- und/oder Untersuchungsbetriebs automatisch erfolgen. Die Verwendung des Laserstrahls zum Erzeugen der Strukturveränderung hat den Vorteil, dass in der Regel die ophthalmologische Vorrichtung nicht um zusätzliche Bauteile ergänzt werden muss.

Nutzt man die Laserstrahlung, um die Strukturveränderung im transparenten Element oder der Fassung zu erzeugen, ist es vorteilhaft, die Strukturveränderung im Inneren des transparenten Elementes oder der (dann transparenten) Fassung zu erzeugen, und nicht an einer Oberfläche, die folglich unverletzt bleibt. Dies erhöht die Sicherheit auch bei irrtümlich versuchter Wiederverwendung, da keine Oberfläche verletzungsträchtig wird

In Ausführungsformen wird die Strukturveränderung mittels einer Umformeinrichtung erzeugt, die optische oder elektrische oder Fluiddichtigkeitseigenschaften modifiziert, indem sie Elemente des Patienteninterfaces mechanisch umformt. Eine Ausführungsform für diese Umformeinrichtung ist ein Körper, der das Patienteninterface umformt, also mechanisch bearbeitet. Dazu hat der Körper eine größere Härte als der Abschnitt des Patienteninterfaces, den er umformt. In einer Ausführungsform wirkt die Umformeinrichtung auf das transparente Element und/oder dessen Fassung. Dabei ist es möglich, dass sie eine Strukturveränderung erzeugt, die die optischen Eigenschaften des Patienteninterfaces ändert. Ein Beispiel hierfür ist eine Ritzung, welche die Transmission von Strahlung beeinflusst oder in einem Bild, das vom entsprechenden Bereich gewonnen wird, optisch detektierbar ist. Ebenfalls ist es möglich, dass die Umformeinrichtung Fluiddichtigkeitseigenschaften des Patienteninterfaces modifiziert, beispielsweise eine für Unterdruckbefestigung benötigte Wandung perforiert.

Die Umformeinrichtung wirkt auf das Patienteninterface dadurch, dass sie mit dieser in Kontakt tritt. Dazu ist eine Relativbewegung zwischen Umformeinrichtung, z. B. dem Körper, und dem Patienteninterface erforderlich. Die Relativbewegung kann durch einen Antrieb zum Bewegen eines umformenden Bauteils, z. B. des Körpers, erzeugt werden, der das Bauteil relativ zum Patienteninterface bewegt. Die Härte des Bauteils ist derart, dass sie das Material des Patienteninterfaces, z. B. das transparente Element und/oder die Fassung, umformen, z. B. ritzen kann. Der Körper kann beispielsweise eine Nadel, einen Stachel, einen Dorn, eine Klinge und/oder einen Bohrer umfassen und das Patienteninterface an einer oder mehreren Stellen umformen. Der Antrieb kann beispielsweise ein Elektromotor oder ein Linearantrieb sein. Er ist optional derart ausgestaltet, dass der Körper lateral und/oder axial über eine Oberfläche des Patienteninterfaces, z. B. des transparenten Elements und/oder der Fassung, bewegt wird. Die Umformeinrichtung ist in Ausführungsformen feststehend, und die Relativbewegung wird von einer Befestigungseinrichtung, mit welcher das Patienteninterface an der ophthalmologischen Vorrichtung angebracht wird, ausgeführt, z. B. indem die Befestigungseinrichtung beim Lösen das Patienteninterface derart vorbewegt wird, dass die Umformeinrichtung in das Patienteninterface, z. B. in das transparente Element und/oder die Fassung, eindringt. Die Befestigungseinrichtung kann beispielsweise einen Bajonettverschluss umfassen, der beim Lösen des Patienteninterfaces von der ophthalmologischen Vorrichtung das Patienteninterface auf die Aufnahme, welche die Umformeinrichtung trägt, hin bewegt. Natürlich kann die Befestigungseinrichtung so ausgebildet sein, dass zusätzlich eine Drehbewegung ausgeführt wird.

In Ausführungsformen weist das transparente Element eine Beschichtung, insbesondere eine Anti-Reflex-Beschichtung, auf, und der Laserstrahl oder die Umformeinrichtung modifizieren die Beschichtung. Die Beschichtung ist insbesondere auf der Seite des transparenten Elements vorgesehen, welche der ophthalmologischen Vorrichtung zugewandt ist. Durch die Modifikation der Beschichtung entstehen Reflektionen oder Streuungen, die einfach detektiert werden können, beispielsweise mithilfe eines Reflexsensors.

Die Umformeinrichtung, welche die Strukturveränderung bewirkt, kann Fluiddichtigkeitseigenschaften modifizieren. Bei der Strukturveränderung, welche die Fluiddichtigkeit modifiziert, weist das Patienteninterface mindestens eine Soll-Bruchstelle auf, welche einen Abschnitt einer fluiddichten Kammer bildet, die das Patienteninterface zusammen mit der ophthalmologischen Vorrichtung und/oder dem Auge bilden kann. Die Soll-Bruchstelle wird beim Anbringen des Patienteninterfaces an der ophthalmologischen Vorrichtung und/oder beim Lösen des Patienteninterfaces von der ophthalmologischen Vorrichtung und/oder zwischen dem Anbringen und dem Lösen des Patienteninterfaces derart zerstört, dass bei einem erneuten Anbringen des Patienteninterfaces an der ophthalmologischen Vorrichtung der Abschnitt nicht mehr fluiddicht ist. Wenn das Patienteninterface von der ophthalmologischen Vorrichtung gelöst wurde, kann es wegen der zerstörten Soll-Bruchstelle nicht mehr verwendet werden. Beispielsweise kann kein Unterdruck in der Kammer mehr aufgebaut werden oder es ist mehr möglich, eine Flüssigkeit in der Kammer zu halten, ohne dass die Flüssigkeit aus der Kammer leckt. Das Zerstören der Soll-Bruchstelle erzeugten eine Undichtigkeit, die einem Benutzer der ophthalmologischen Vorrichtung unmittelbar ersichtlich ist, sodass zuverlässig sichergestellt ist, dass ein Patienteninterface, das zum einmaligen Gebrauch vorgesehen ist, nicht irrtümlich ein zweites Mal verwendet wird. In entsprechenden Ausführungsformen umfasst das Patienteninterface eine Unterdruckkammer, mittels welcher das Patienteninterface durch Unterdruck an der ophthalmologischen Vorrichtung angebracht wird, und/oder einen Saugkanal, der das Auge per Unterdruck an das Patienteninterface saugt, und/oder eine Flüssigkeitskammer zwischen dem Auge und dem Patienteninterface. Beispielsweise bilden das Patienteninterface und die Aufnahme zusammen eine Unterdruckkammer. Dies bedeutet, dass das Patienteninterface einerseits und die ophthalmologische Vorrichtung andererseits jeweils unterdruckdichte Flächen aufweisen, die durch das Anbringen des Patienteninterfaces an der ophthalmologischen Vorrichtung zusammen die Unterdruckkammer vervollständigen. Durch Anlegen von Unterdruck an die Unterdruckkammer wird das Patienteninterface an der ophthalmologischen Vorrichtung gehalten. Ferner ist es möglich, dass eine Flüssigkeitskammer vorgesehen ist, um Flüssigkeit zwischen das Auge und das Patienteninterface einzubringen. Dies ist bei manchen Patienteninterfaces vorteilhaft, da damit eine Brechungsindexfehlanpassung zwischen Auge und/oder Patienteninterface verringert werden kann. In diesem Fall bildet das Patienteninterface eine Wand der Flüssigkeitskammer. In die Flüssigkeitskammer wird Flüssigkeit eingebracht. Ferner ist es auch möglich, dass über einen Saugkanal ein Unterdruck erzeugt wird, mittels welchem das Patienteninterface am Auge fixiert wird.

Für diese Ausführungsformen weist die Umformeinrichtung der ophthalmologischen Vorrichtung eine Durchstoßeinrichtung auf, die an der ophthalmologischen Vorrichtung angeordnet ist und von der ophthalmologischen Vorrichtung in Richtung des Patienteninterfaces vorsteht. Die Durchstoßeinrichtung kann eine oder mehrere Nadeln, Dorne, Stacheln, Pfeile und/oder eine Schneidkante aufweisen. Die Durchstoßeinrichtung kann beispielsweise aus Metall gefertigt sein. Die Durchstoßeinrichtung ist härter als die Wandung an der Soll-Bruchstelle. Sie durchtrennt eine Wandung der Unterdruckkammer, der Flüssigkeitskammer und/oder des Saugkanals, so dass eine Undichtigkeit erzeugt wird. Die Durchstoßeinrichtung wirkt mit einer Soll-Bruchstelle im Patienteninterface zusammen, die an der Stelle der Wandung sitzt, an der die Durchstoßeinrichtung die Wandung perforiert. In Ausführungsformen ist an der Soll-Bruchstelle die Wandung elastisch ausgebildet. Die elastische Wandung hat die Wirkung, dass die Soll-Bruchstelle weiterhin fluiddicht ist, solange die Durchstoßeinrichtung in der Wandung sitzt. Die Soll-Bruchstelle kann beispielsweise eine Gummi- oder Silikonmembran sein, die sich fluiddicht an der Durchstoßeinrichtung anlegt. Die von der Durchstoßeinrichtung durchstoßene Wandung kann besonders bevorzugt in der Fassung des transparenten Elementes sitzen, welche als trennende Wandung zwischen der Unterdruckkammer, mittels welcher das Patienteninterface an der Aufnahme befestigt wird, und dem Saugkanal, über den das Patienteninterface am Auge angesaugt wird, sitzt. Alternativ kann die Wandung auch das trennende Element zwischen der genannten Unterdruckkammer und der Flüssigkeitskammer oder zwischen dem Saugkanal und der Flüssigkeitskammer sein. Die Durchstoßeinrichtung wird mit einer Relativbewegung zwischen Patienteninterface und Aufnahme durch die Wandung getrieben, wobei die Relativbewegung wiederum entweder durch eine entsprechend ausgestaltete Befestigungseinrichtung beim Anbringen des Patienteninterfaces an der Aufnahme bewirkt wird, oder durch einen separaten, die Durchstoßeinrichtung vorschiebenden Antrieb. Nach dem Lösen des Patienteninterfaces von der ophthalmologischen Vorrichtung weist das Patienteninterface im Durchstoßbereich Durchgänge aufgrund der Durchstoßeinrichtung auf, welche das Patienteninterface nicht mehr fluiddicht machen. Auf diese Weise kann das Patienteninterface nicht wiederverwendet werden. Diese Weiterbildung kann bautechnisch einfach implementiert werden, und das Eindringen der Durchstoßeinrichtung in den Durchstoßbereich bewirkt zudem eine zusätzliche Fixierung des Patienteninterfaces an der ophthalmologischen Vorrichtung. Das Eindringen der Durchstoßeinrichtung in die Soll-Bruchstelle erfolgt vorzugsweise beim Anbringen des Patienteninterfaces an der ophthalmologischen Vorrichtung, kann aber auch während des Lösens geschehen.

In Ausführungsformen weist das Patienteninterface mindestens einen Fluidkanal, z. B. einen Schlauch, zur Verbindung mit einer Unterdruckquelle oder einem Flüssigkeitsreservoir der ophthalmologischen Vorrichtung auf, wobei der Kanal eine Soll-Bruchstelle aufweist. Die Soll-Bruchstelle wird beim Verbinden des Kanals mit einem Anschluss an das Flüssigkeitsreservoirs bzw. die Unterdruckquelle und/oder beim Lösen der Verbindung zerstört. Die Unterdruckquelle kann beispielsweise eine Saugpumpe sein, welche Unterdruck für die Unterdruckkammer oder den Saugkanal erzeugt. Das Reservoir stellt die Flüssigkeit für die Flüssigkeitskammer bereit. Der Fluidkanal kann beispielsweise aus Kunststoff hergestellt sein und ein Schlauch sein. Vorteil dieser Ausführungsformen ist es, dass die Zerstörung der Soll-Bruchstelle nicht in unmittelbarer Nähe des Auges erfolgt, sondern nahe der ophthalmologischen Vorrichtung. Damit lassen sich potenzielle Gefährdungen des Auges vermeiden. In einer Ausführungsform ist es vorgesehen, dass der Anschluss an der Vorrichtung eine Schneideinrichtung umfasst, welche die Soll-Bruchstelle des Fluidkanals zerstört. Z. B. kann der Anschluss eine Schneidkante aufweisen, welche beim Lösen des Fluidkanals vom Anschluss die Soll-Bruchstelle zerstört. In Ausführungsformen ist am Anschluss eine Schneidkante fixiert und beispielsweise so angeordnet, dass sie beim Lösen des Fluidkanals vom Anschluss den Kanal und/oder eine Dichtung anritzt oder einschneidet. Ferner kann die Schneidkante beweglich angeordnet sein und beim Lösen des Fluidkanals vom Anschluss ausfahren, wodurch der Kanal geschnitten oder angeritzt wird. Es ist auch eine Ausführungsform möglich, die den Fluidkanal beim Anbringen so modifiziert, dass im angeschlossenen Zustand noch Dichtigkeit besteht, diese nach dem Lösen aber nicht mehr herstellbar ist. Beispielsweise schneidet ein Gewinde in ein Ende des Fluidkanals und/oder verformt es. Besonders bevorzugt ist ein Einwegverbinder, der nur einmal angeschlossen werden kann und ein erneutes Anschließen unmöglich macht.

In einer weiteren Ausführungsform erfolgt die Strukturveränderung an einer Soll-Bruchstelle die derart ausgebildet, dass sie oberhalb eines vorgegebenen Differenzdrucks bricht. Vor dem Lösen des Patienteninterfaces wird der Druck in der Kammer, welche die Soll-Bruchstelle aufweist, so eingestellt, dass die Soll-Bruchstelle bricht. Das Lösen des Patienteninterfaces umfasst dann diese Druckänderung. Beispielsweise ist die Soll-Bruchstelle als sich von der restlichen Wandung unterscheidende dünnwandige Membran ausgestaltet, die einem regulären Druck zwischen dem Patienteninterface und der ophthalmologischen Vorrichtung oder zwischen dem Patienteninterface und dem Auge standhält. Wird der Druck derart abgesenkt, dass der vorgegebene Differenzdruck überschnitten wird, bricht die Soll-Bruchstelle. Beispielsweise kann der Benutzer der ophthalmologischen Vorrichtung anzeigen, dass er das Patienteninterface lösen möchte. In diesem Fall kann die ophthalmologische Vorrichtung automatisch den Unterdruck erhöhen, bis die Soll-Bruchstelle bricht. Auf diese Weise wird zugleich der Unterdruck in der Unterdruckkammer oder am Saugkanal abgebaut.

Weitere Ausführungsformen sind darauf gerichtet, dass ein Patienteninterface ein transparentes Element aufweist, durch welches bei einem Betrieb der ophthalmologischen Vorrichtung Strahlung zur Beobachtung und/oder Behandlung eines Auges eines Patienten läuft. Sie nutzen weiter aus, dass das Patienteninterface vor einer Verwendung steril sein muss. Bei der Erstverwendung ist die Sterilität durch die werksseitige Sterilisierung sichergestellt. Bei einer Wiederverwendung müsste eine nochmalige Sterilisierung oder eine Desinfektion erfolgen. Um ein solches in der Regel suboptimales Vorgehen und damit eine Wiederverwendung des Patienteninterfaces klar erkennbar zu machen, weist das transparente Element eine reaktive Schicht auf, die bei einer bestimmten Sterilisierung oder Desinfektion des Patienteninterfaces eine optische Eigenschaft ändert. Die weitere Möglichkeit zum Sicherstellen der Sterilität eines Patienteninterfaces der genannten Art, das an einer ophthalmologischen Vorrichtung eingesetzt wird, besteht also darin, das genannte transparente Element des Patienteninterfaces auf bestimmte Weise auszugestalten. Durch das transparente Element läuft im Betrieb der Vorrichtung Strahlung zum Auge des Patienten. Die bestimmte Ausgestaltung besteht darin, dass das transparente Element mit einer reaktiven Schicht versehen wird. Diese nimmt eine vorbestimmte optische Eigenschaft bei einer vorbestimmten Sterilisierung oder Desinfektion des Patienteninterfaces an. Hierunter fällt eine Zweitsterilisierung des Patienteninterfaces, also eine Wiederholung der bereits werksseitig vorgenommenen Sterilisierung oder eine andere Sterilisierungs- oder Desinfektionsmethode, als sie werksseitig eingesetzt wird. Bei der Herstellung eines Patienteninterfaces wird üblicherweise eine Sterilisierung mit ionisierender Strahlung eingesetzt. Die vorbestimmte Sterilisierung umfasst deshalb eine zweite Verwendung ionisierender Strahlung zur Sterilisierung oder eine chemische Sterilisierung oder eine Dampfsterilisierung oder eine Wischdesinfektion. Dann nimmt die reaktive Schicht die vorbestimmte optische Eigenschaft an. Sie ändert beispielsweise ihre Transparenz und wird opak. Eine solche Änderung kann besonders einfach detektiert werden und zeigt eine Wiederverwendung des Patienteninterfaces an. Dann wird der Untersuchungs- und/oder Behandlungsbetrieb der Vorrichtung blockiert. Es wird überprüft, ob die reaktive Schicht diese optische Eigenschaft aufweist; falls ja, wird ein weiterer Betrieb der ophthalmologischen Vorrichtung gestoppt, insbesondere ist es nicht möglich, das Auge zu beobachten und/oder zu behandeln. Da das Patienteninterface herstellerseitig in der Regel mit ionisierender Strahlung sterilisiert wird, ist es einfach, die reaktive Schicht daraufhin auszulegen, dass chemische Sterilisierung oder Dampfsterilisierung zur gewünschten Änderung der optischen Eigenschaft führt. Gleichermaßen ist es möglich, die reaktive Schicht so zu gestalten, dass sie bei einer zweiten Sterilisierung mit ionisierender Strahlung die Eigenschaftsänderung ausführt, nicht jedoch bei der herstellerseitigen ersten Sterilisierung mit ionisierender Strahlung. Die reaktive Schicht ist vor der bei der Wiederverwendung durchgeführten Sterilisierung oder Desinfektion transparent, sodass Strahlung zur Beobachtung und/oder Behandlung durch die reaktive Schicht nicht gestört wird, und nimmt bei erneuter Sterilisierung oder einer Desinfektion eine optische Eigenschaft derart an, dass sie dann mit der Strahlung im Hinblick auf Beobachtung und/oder Behandlung störend wechselwirkt. Die Änderung der optischen Eigenschaft kann beispielsweise eine Verfärbung, Opazität oder Änderung der Transparenz oder des Streu- oder Reflexionsverhaltens sein. Es ist dann unmittelbar ersichtlich, ob die reaktive Schicht die optische Eigenschaft annahm. Insbesondere ist es mit der optischen Eigenschaft der reaktiven Schicht nicht mehr möglich, die Strahlung zur Behandlung und/oder Beobachtung des Auges durch die reaktive Schicht zu führen. Vorzugsweise erstreckt sich die reaktive Schicht über den ganzen Bereich des transparenten Elements, durch welchen Strahlung zur Beobachtung und/oder Behandlung geführt wird. Vorteil dieser Ausführungsformen ist es, dass mit der weiteren Desinfektion des Patienteninterfaces eine gut erkennbare Markierung, dass dieses Patienteninterface bereits verwendet wurde, entsteht. So weist die reaktive Schicht zuverlässig nach, dass das für den nur einmaligen Gebrauch vorgesehene Patienteninterface bereits verwendet wurde. In einer optionalen Weiterbildung ändert die reaktive Schicht beim Erwärmen auf eine vorgegebene Temperatur und/oder bei Kontakt mit einem Desinfektionsfluid die optische Eigenschaft. Die vorgegebene Temperatur liegt insbesondere unterhalb der Temperatur für die Hitzebehandlung, welche im Zuge eines Autoklavier-Verfahrens erfolgt. Ferner kann die Sterilisation eine Behandlung mit UV- oder Röntgenstrahlung umfassen, welche zu einer lichtinduzierten Reaktion in der reaktiven Schicht führt. Darüber hinaus kann die Desinfektion eine Wischdesinfektion mit einem Desinfektionsfluid umfassen, welches beim Kontakt mit der reaktiven Schicht dessen optischen Eigenschaften verändert. Da die Sterilisierung durch den Hersteller in der Regel in einer Transportverpackung durch ionisierende Strahlung erfolgt, ist es bevorzugt vorgesehen, dass die reaktive Schicht die geänderten optischen Eigenschaften nur annimmt, wenn eine Sterilisierung oder Desinfektion außerhalb einer Transportverpackung erfolgt, in welcher das Patienteninterface vom Hersteller geliefert wird.

Alternativ zur Änderung optischer Eigenschaften kann eine Strukturänderung eingesetzt werden, z. B. indem das Patienteninterface ein Material umfasst, das bei einer Autoklavierung erweicht.

Es sind im Stand der Technik Patienteninterfaces bekannt, die einen Funkchip (z. B. RFID-Chip) enthalten, welcher Daten über das Patienteninterface speichert. Im einfachsten Fall sind diese Daten eine Identifikationsnummer des Funkchips oder Patienteninterfaces. Gleichermaßen ist es bekannt, Typangaben über das Patienteninterface in einem Funkchip abzuspeichern. Solche Typangaben können optische oder geometrische Eigenschaften des Patienteninterfaces angeben. Ein solcher Funkchip wird vor der eigentlichen Untersuchung/Behandlung funkbasiert ausgelesen. Bei solchen Patienteninterfaces ist eine Sicherstellung der Sterilität nun dadurch vorgesehen, dass der Funkchip nach dem erforderlichen Auslesen zerstört wird. Dies kann durch mechanische Einwirkung auf den Funkchip oder unter Einsatz von Laserstrahlung erfolgen. Es genügt dabei, den Funkchip funktionsunfähig zu machen, beispielsweise eine Antenne oder Antennenleitung, die der Funkchip für die Kommunikation benötigt, zu zerstören oder zu durchtrennen. Dies kann mittels Laserstrahlung oder der genannten mechanischen Umformeinrichtung erfolgen.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen. Insbesondere ist es möglich, die Merkmale einzelner Ausführungsformen zu kombinieren, um das Risiko einer Wiederverwendung des Patienteninterfaces weiter zu reduzieren.

Nachfolgend wird die Erfindung beispielsweise anhand der beigefügten Zeichnungen, die auch erfindungswesentliche Merkmale offenbaren, noch näher erläutert. Es zeigen:
- Fig. 1A und 1B: zwei Varianten einer ersten Ausführungsform eines Patienteninterfaces,
- Fig. 2A und 2B: zwei Varianten einer zweiten Ausführungsform eines Patienteninterfaces,
- Fig. 3A und 3B: zwei Varianten einer dritten Ausführungsform eines Patienteninterfaces,
- Fig. 4A und 4B: zwei Varianten einer vierten Ausführungsform eines Patienteninterfaces,
- Fig. 5A und 5B: zwei Varianten einer fünften Ausführungsform eines Patienteninterfaces und
- Fig. 6: eine sechste Ausführungsform eines Patienteninterfaces.

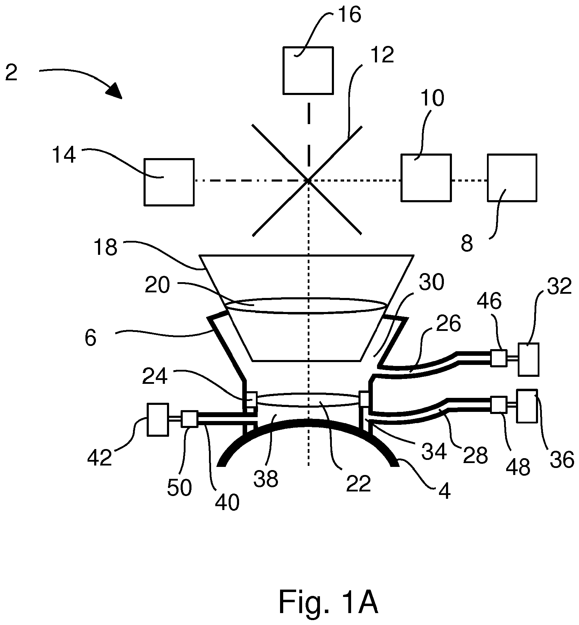

Eine ophthalmologische Vorrichtung 2 dient zur Behandlung und/oder Beobachtung eines Auges 4 eines Patienten. Dazu ist, wie Fig. 1A zeigt, ein Patienteninterface 6 vorgesehen, das als Schnittstelle zwischen der ophthalmologischen Vorrichtung 2 und dem Auge 4 dient. Das Patienteninterface 6 ist ein optisches Element eines Strahlenganges zur Beobachtung und/oder Behandlung des Patientenauges. Es bewirkt zugleich eine mechanische Fixierung des Auges 4 gegenüber der ophthalmologischen Vorrichtung 2. Die Fixierung des Auges 4 ist hilfreich, um es präzise beobachten/behandeln zu können.

Die ophthalmologische Vorrichtung 2 weist optional eine Laserquelle 8, eine Laserstrahlführeinrichtung 10, einen Strahlteiler 12, eine OCT-Einrichtung 14, ein Operationsmikroskop 16, eine Aufnahme 18 und/oder eine Linse 20 auf.

Für die ophthalmologische Vorrichtung 2 gibt es verschiedene Ausführungsformen. In einigen Ausführungsformen dient die ophthalmologische Vorrichtung 2 der Beobachtung des Auges 4. Sie weist dazu beispielsweise die OCT-Einrichtung 14 und/oder das Operationsmikroskop 16 auf. In anderen Ausführungsformen ist die ophthalmologische Vorrichtung 2 zur Behandlung des Auges mittels Behandlungslaserstrahlung ausgebildet und weist hierzu beispielsweise die Laserquelle 8 auf. Die optionale Laserquelle 8 ist bevorzugt ausgebildet, Koagulation, Photoablation, plasmagestützte Ablation oder Photodisruptionen im Auge 4, beispielsweise an der Retina, im Bereich der Augenlinse oder in der Hornhaut, vorzunehmen. Sie umfasst beispielsweise einen Femtosekundenlaser. Der von der Laserquelle 8 bereitgestellte Laserstrahl (in den Figuren kurz gestrichelt eingezeichnet) wird von der Laserstrahlführeinrichtung 10 abgelenkt. Die Laserstrahlführeinrichtung 10 kann beispielsweise einen oder mehrere Scanner zur lateralen Fokusverschiebung und eine Optik zur Veränderung der Lage des Fokus entlang der Propagationsrichtung des Laserstrahls umfassen. Die optionale OCT-Einrichtung 14 ist ausgebildet, ein OCT-Bild, beispielsweise der Augenlinse des Auges 4, aufzunehmen. Der Strahlengang, durch den sie angekoppelt ist, ist strichpunktiert gezeichnet. Das optionale Operationsmikroskop 16 dient zur Abbildung von Strukturen des Auges 4, z. B. der Iris oder der Retina. Seine Strahlenganganbindung ist langgestrichelt symbolisiert.

Strahlung zur Beobachtung und/oder Behandlung des Auges 4 wird mittels des Strahlteilers 12 zur Linse 20 und über ein transparentes Element 22, z. B. ebenfalls eine Linse, in das Auge 4 geführt. Die Aufnahme 18 ist in der Regel das Bauteil der ophthalmologischen Vorrichtung 2, welches dem Auge 4 am nächsten liegt. Sie dient zum Anbringen des Patienteninterfaces 6 an der ophthalmologischen Vorrichtung 2.

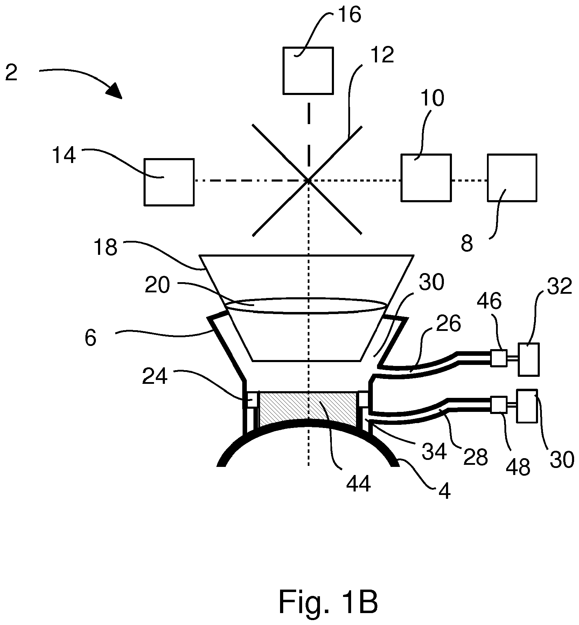

Das Patienteninterface 6 weist das transparente Element 22, eine dieses haltende Fassung 24, und in der Ausführungsform der Figur 1A und 1B zwei Unterdruckleitungen 26, 28 auf. Das transparente Element 22 dient zum Führen der Strahlung von und zum Auge 4. Z. B. wird der Laserstrahl der Laserquelle 8 u.a. durch das transparente Element 22 fokussiert. Die Fassung 24 kann ebenfalls transparent ausgestaltet sein. Optional ist sie intransparent oder opak. Durch die Fassung 24 läuft keine Strahlung zur Behandlung und/oder Untersuchung. Das transparente Element 22 und die Fassung 24 teilt das Patienteninterface 6 horizontal (bezogen auf die Zeichnung). Oberhalb befindet sich eine Unterdruckkammer 30, welche durch das transparente Element 22 und die Fassung 24 sowie weiter durch die Aufnahme 18 und die Wandung des Patienteninterfaces 6 begrenzt ist. Die Unterdruckkammer 30 wird über die Unterdruckleitung aus einer Unterdruckquelle, z. B. einer Saugpumpe 32, evakuiert. Zum Anbringen des Patienteninterfaces 6 an der ophthalmologischen Vorrichtung 2 wird so in der Unterdruckkammer 30 Unterdruck erzeugt und das Patienteninterface 6 per Vakuum fixiert. Zum Lösen des Patienteninterfaces 6 von der ophthalmologischen Vorrichtung 2 wird die Unterdruckkammer 30 belüftet.

Unterhalb (wiederum bezogen auf die Zeichnung) der Unterdruckkammer 30 befindet sich ein ringförmiger Saugkanal 34. Er dient dem Ansaugen des Patienteninterfaces 6 am Auge 4 und ist über die Unterdruckleitung 28 mit einer weiteren Saugpumpe 36 verbunden. Die beiden Saugpumpen 32, 36 können auch durch unterschiedliche Kreise einer einzigen Saugpumpe ersetzt werden.

Weiter ist zwischen Element 22 und der Vorderseite des Auges 4 eine Flüssigkeitskammer 38 gebildet, wenn das Patienteninterface 6 an dem Auge 4 anliegt. Sie wird vom ringförmigen Saugkanal 34 umgeben. In die Flüssigkeitskammer 38 wird über eine Zuleitung 40 eine Flüssigkeit aus einem Reservoir 42 eingeführt, um Reflexionen an Luft/Material-Übergängen zu vermeiden und einen Brechungsindex-Missmatch zwischen dem transparenten Element 22 der Flüssigkeitskammer 38 und dem Auge 4 und zu mindern.

Zum Anbringen des Patienteninterfaces 6 an der ophthalmologischen Vorrichtung 2 wird zuerst das Patienteninterface 6 an die Aufnahme 18 angelegt und Unterdruck in der Unterdruckkammer 30 erzeugt. Nun ist das Patienteninterface 6 an der ophthalmologischen Vorrichtung 2 fest fixiert. Zum Anbringen des Patienteninterfaces am Auge wird das Patienteninterface 6 auf die Vorderseite des Auges 4 aufgesetzt und über die Unterdruckleitung 28 am Saugkanal 34 angesaugt. Anschließend wird die Flüssigkeitskammer 38 zwischen der Vorderseite des Auges 4 und dem transparenten Element 22 durch die Zuleitung 40 aus dem Reservoir 42 mit einer Flüssigkeit befüllt, die den Brechungsindex zwischen transparentem Element 22 und Auge 4 anpasst. Die Reihenfolge, in welcher das Patienteninterface am Auge 4 und der ophthalmologischen Vorrichtung 2 befestigt wird, ist prinzipiell frei wählbar. In bestimmten Ausführungsformen hat es sich jedoch bewährt, das Patienteninterface 6 zuerst an der ophthalmologischen Vorrichtung 2 anzubringen, da bei der dann folgenden Befestigung am Auge 4 Mittel der ophthalmologischen Vorrichtung 2 zum Justieren der richtigen Lage des Patienteninterfaces 6 am Auge 4 genutzt werden können, beispielsweise das Operationsmikroskop und/oder die OCT-Einrichtung.

Das Patienteninterface 6 befindet sich ersichtlich in direktem Kontakt mit dem Auge 4. Es muss also auf ausreichende Hygiene geachtet werden. Das Patienteninterface 6 ist deshalb als steriles Einwegverbrauchsmittel ausgelegt. Eine mehrmalige Verwendung des Patienteninterfaces 6 würde Hygieneanforderungen zuwider laufen. Es ist deshalb, wie im allgemeinen Teil der Beschreibung bereits erläutert wurde und nachfolgend anhand von Ausführungsbeispielen noch näher ausgeführt werden wird, vorgesehen, dass im Zusammenhang mit der erstmaligen Verwendung eines Patienteninterfaces 6 eine Strukturveränderung erzeugt wird. Sie stellt entweder eine Entwertungsmarkierung dar, die anzeigt, dass das Patienteninterface 6 bereits benutzt wurde, oder macht das Patienteninterface 6 so unbrauchbar, dass eine Wiederverwendung technisch unmöglich ist.

Anhand der Entwertungsmarkierung kann die ophthalmologische Vorrichtung 2 eigenständig feststellen, ob versehentlich ein Patienteninterface 6 angebracht wurde, das bereits benutzt worden ist. Auf diese Weise ist sichergestellt, dass ein Patienteninterface 6 an der ophthalmologischen Vorrichtung 2 nur einmal verwendet werden kann. Dazu überprüft die ophthalmologische Vorrichtung 2, ob bereits eine Entwertungsmarkierung am Patienteninterface 6, z. B. am transparenten Element 22 oder der Fassung 32, vorhanden ist; falls ja, wird ein weiterer Betrieb der ophthalmologischen Vorrichtung 2 verhindert. Beispielsweise erzeugt die Laserquelle 8 dann keinen Laserstrahl und/oder die OCT-Einrichtung 14 nimmt kein OCT-Bild auf. Wird keine Entwertungsmarkierung am Patienteninterface 6 festgestellt, kann die ophthalmologische Vorrichtung 2 zum Untersuchen, Beobachten und/oder Behandeln des Auges 4 bestimmungsgemäß benutzt werden.

Falls keine Entwertungsmarkierung aufgefunden wurde, was üblicherweise den Regelfall darstellt, ist natürlich sicherzustellen, dass das Patienteninterface 6, welches aktuell in Einsatz ist oder in Einsatz gebracht werden soll, noch mit einer Entwertungsmarkierung versehen wird, um eine Wiederverwendung auszuschließen. Es ist deshalb vorgesehen, dass die ophthalmologische Vorrichtung 2 eine entsprechende Entwertungsmarkierung anbringt. Der Zeitpunkt, zu dem die Entwertungsmarkierung erzeugt wird, ist je nach Ausführungsform frei wählbar. Es ist natürlich darauf zu achten, dass die ophthalmologische Vorrichtung 2 nicht irrtümlich eine gerade erst erzeugte Entwertungsmarkierung erfasst und den weiteren bestimmungsgemäßen Betrieb der Vorrichtung sperrt. Dies kann auf zwei Arten erreicht werden. In einer Ausführungsform ist es vorgesehen, dass vor dem Erzeugen der Entwertungsmarkierung geprüft wird, ob bereits eine Entwertungsmarkierung vorhanden ist. Eine irrtümliche Detektion einer in der aktuellen Verwendung erzeugten Entwertungsmarkierung ist damit ausgeschlossen. In einer alternativen Ausführungsform ist die Entwertungsmarkierung so gestaltet, dass es ausgeschlossen ist, dass bei einer Wiederverwendung exakt dieselbe Entwertungsmarkierung erzeugt wird. Da es bekannt ist, wo und wie die Entwertungsmarkierung der aktuellen Verwendung ausgeführt ist, steht eine irrtümliche Detektion der in der aktuellen Verwendung erzeugten Entwertungsmarkierung nicht zu befürchten.

In anderen Ausführungsformen macht die Strukturveränderung das Patienteninterface 6 unbrauchbar. Hier ist in Ausführungsformen die Strukturveränderung so ausgeführt, dass, solange das Patienteninterface noch nicht von der Aufnahme gelöst wird, auch mit Strukturveränderung ein bestimmungsgemäßer Einsatz des Patienteninterfaces 6 mit ophthalmologischer Vorrichtung 2 noch möglich ist, ein nochmaliges Anbringen des Patienteninterfaces 6 an der ophthalmologischen Vorrichtung 2 und/oder dem Auge 4 jedoch aus technischen Gründen nicht möglich ist. Beispiele für solche Entwertungsmarkierungen wurden bereits im allgemeinen Teil der Beschreibung erläutert und werden nachfolgend in Ausführungsbeispielen noch näher geschildert. Ein besonders eingängiges Beispiel ist die Verwendung eines Dorns, der eine Wandung der Unterdruckkammer durchstößt, nachdem das Patienteninterface 6 per Unterdruck an der Aufnahme 18 angebracht wurde, und der so dichtend in der Wandung sitzt, dass die Unterdruckkammer 30 weiterhin arbeitet. Erst beim Lösen des Patienteninterfaces 6 von der Aufnahme 18 bleibt die erzeugte Durchstoßöffnung in der Wandung, so dass die Unterdruckkammer beim nochmaligen Versuch, dasselbe Patienteninterface 6 an der Aufnahme 18 zu befestigen, nicht mehr dicht wäre. Das Patienteninterface 6 lässt sich dann kein zweites Mal an der Aufnahme 18 per Unterdruck ansaugen. Dies stellt jedoch nicht die einzige Variante dar; sie sei hier nur angeführt, um einen ersten Überblick über das Grundprinzip zu erhalten, dass das Patienteninterface 6 bei der Erstbefestigung an der Aufnahme 18 so modifiziert wird, dass eine nochmalige Befestigung desselben Patienteninterfaces 6 aus technischen Gründen nicht möglich ist. Es bedarf keiner weiteren Erläuterung, dass eine Variante, die bei der Erstbefestigung des Patienteninterfaces 6 an der Aufnahme 18 das Patienteninterface 6 so modifiziert, dass eine Wiederbefestigung nicht mehr möglich ist, keinen expliziten Detektionsschritt benötigt, ob eine Strukturveränderung vorhanden ist. Die Strukturveränderung modifiziert das Patienteninterface 6 ja so, dass eine Wiederbefestigung ausscheidet. Eine separate Überprüfung, ob eine Erstbefestigung bereits stattgefunden hat, erübrigt sich.

In der in Figur 1A dargestellten Ausführungsform dient die ophthalmologische Vorrichtung 2 sowohl der Beobachtung als auch der Behandlung. In einigen Ausführungsformen, in denen die ophthalmologischen Vorrichtung 2 die Laserquelle 8 und Laserstrahlführeinrichtung 10 aufweist, ist es vorgesehen, dass die Laserquelle 8 zur Erzeugung der Strukturveränderung eingesetzt wird. Dabei wird in Ausführungsformen die Strukturveränderung als Entwertungsmarkierung in das transparente Element 22 und/oder die Fassung 24 eingebracht. Die Laserstrahlführeinrichtung 18 richtet dazu den Laserstrahl auf das transparente Element 22 und/oder die Fassung 24. Beispielsweise werden Teile der Fassung und/oder des transparenten Elements 22, insbesondere eine Beschichtung, wie eine Anti-Reflex-Beschichtung, zerstört. Die Entwertungsmarkierung kann beispielsweise ein eingebrachtes Muster, einen Strichcode und/oder einen Schriftzug umfassen. Der Schriftzug und/oder der Strichcode, wie ein Barcode, können einen Zeitstempel, den Namen des Patienten, den Namen des behandelten Arztes oder dergleichen angeben. Die Entwertungsmarkierung kann beispielsweise konzentrisch eingebracht sein oder radiale Strukturen umfassen. Mögliche Schnittmuster in dem transparenten Element 22 können Kreuzschnitte, Kreisschnitte, Flächenschnitte oder Kombinationen aus Rund- und Linienschnitten sein. Die Veränderung erfolgt bevorzugt im Inneren des Elementes 22 oder der Fassung 24, also unter der Oberfläche des Bauteils. Optional ist die Entwertungsmarkierung so groß, dass aufgrund von Streueffekten das transparente Element 22 nicht weiter als optisches Bauteil tauglich ist. Die Entwertungsmarkierung erzeugt z. B. Streuungen in dem transparenten Element 22, sodass die Fokusgüte unzulässig abnimmt und z. B. keine Mehrphotonenabsorption mehr stattfinden kann. Sie macht dann auch das Patienteninterface unbrauchbar.

Zum Feststellen, ob eine Entwertungsmarkierung vorhanden ist, wird ein Bild des transparenten Elements 22 aufgenommen. Hierzu kann das Operationsmikroskop 16, und/oder die OCT-Einrichtung 14 und/oder eine zusätzliche Kamera eingesetzt werden. Wenn die Entwertungsmarkierung eine Anti-Reflex-Beschichtung auf einer der ophthalmologischen Vorrichtung 2 zugewandten Seite des transparenten Elements 22 abträgt, werden Reflexe an der Entwertungsmarkierung detektiert.

Die Strukturveränderung kann auch in der Fassung 32 erzeugt werden. Eine sichtbare Entwertungsmarkierung in der Fassung 32 kann zur Kalibration der Laserstrahlführeinrichtung 18 verwendet werden. In diesem Fall wird die Entwertungsmarkierung noch vor der Beobachtung und/oder Behandlung des Auges 4, aber natürlich nach der Überprüfung auf Wiederverwendung, eingebracht.

Figur 1B zeigt eine Abwandlung des Aufbaus der Figur 1A. Hier ist das transparente Element als Kontaktglas 44 ausgebildet, welches an die Vorderfläche des Auges 4 angepresst wird und so der Vorderfläche der Hornhaut des Auges 4 eine gewünschte Krümmung verleiht. So entfallen alle Merkmale in der Ausführungsform der Figur 1A, die mit der Flüssigkeitskammer 38 in Verbindung stehen.

Bei den Aufbauten gemäß Figur 1A und 1B sind auch Ausführungsformen möglich, welche die Entwertung des Patienteninterfaces 6 nicht durch eine Modifikation des transparenten Elementes 22, 44 oder dessen Fassung 24 bewirken, sondern durch eine Modifikation einer Fluidverbindung, über welche Unterdruck angelegt oder Flüssigkeit zugeführt wird. Der Begriff "Fluid" wird hier im üblichen technischen Verständnis verwendet, umfasst also sowohl Flüssigkeiten als auch Gase. In einer Ausführungsform ist mindestens eine der Unterdruckleitungen 26, 28 und/oder die Zuleitung 40 mit einem Verbinder 46, 48, 50 versehen, der als Einwegverbinder ausgebildet ist. Auf diese Weise ist sichergestellt, dass Unterdruck in der Unterdruckkammer 30 und/oder im Saugkanal 34 und/oder das Zuführen der Flüssigkeit in die Flüssigkeitskammer 38 nur bei Erstverwendung des Patienteninterfaces 6 möglich ist. Fluid-Einwegverbinder sind dem Fachmann sowohl für Druckleitungen, also für die Verbinder 46 und 48, als auch für Flüssigkeitsleitungen, also für den Verbinder 50, bekannt. Sie weisen beispielsweise eine Soll-Bruchstelle auf, die beim Lösen des Verbinders bricht und ein nochmaliges Anschließen nicht zulässt. In einer anderen Ausführungsform kann der entsprechende Anschluss zur Saugpumpe 32, 36 und/oder dem Reservoir 42 mit einer Schneideinrichtung versehen sein, welche beim Lösen eine Soll-Bruchstelle zerstört. Beispielsweise kann die Schneideinrichtung so angeordnet sein, dass beim Abziehen die Leitung 26, 28, 40 eingeritzt oder zerschnitten wird. Z. B. kann eine ausfahrbare Schneidklinge beim Abziehen ausfahren und die Leitung oder den Steckverbinder zerstören. In Ausführungsformen ist die Leitung 26, 28, 40 mittels einer Fixiereinrichtung befestigt, welche eine Soll-Bruchstelle und/oder die Leitung zerstört - aber, solange sie angebracht ist, den Anschluss der Leitung fluiddicht hält. Eine solche Fixiereinrichtung kann beispielsweise als Mutter ausgebildet sein, deren Gewinde eine Soll-Bruchstelle und/oder Leitung einschneidet und/oder verformt.

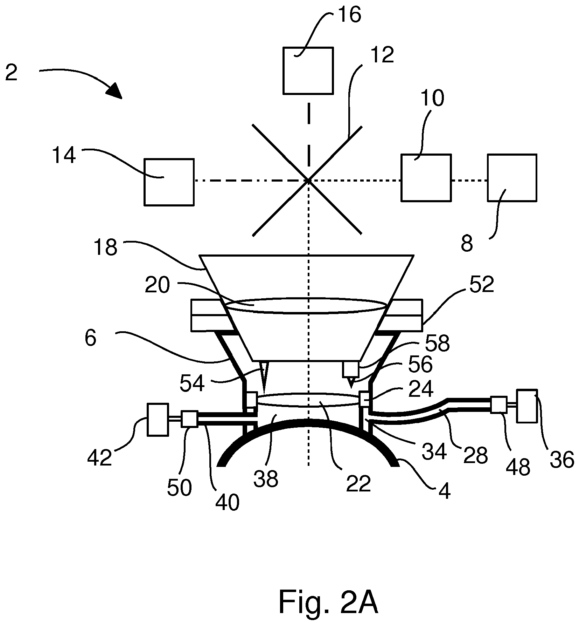

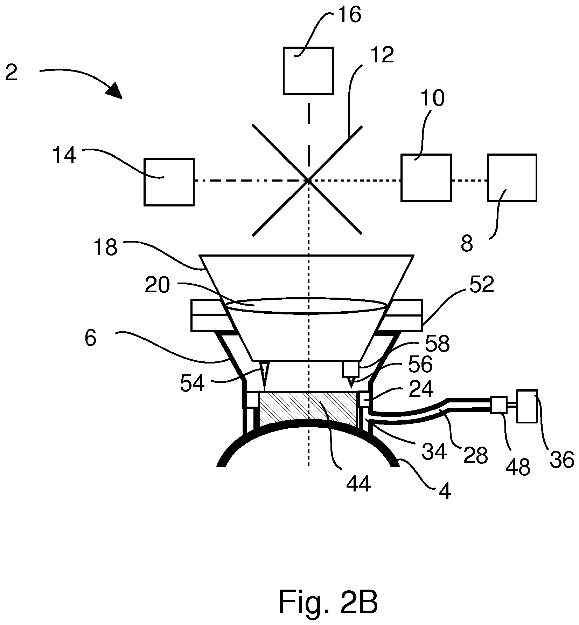

Bei den in Figuren 2A und 2B dargestellten Ausführungsformen hat die ophthalmologische Vorrichtung 2 abweichend von den Figuren 1A und 1B eine Umformeinrichtung, hier u. a. umfassend eine Befestigungseinrichtung 52 und einen Körper 54 oder 56 zum Umformen des Patienteninterfaces 6. In diesen Ausführungsformen sorgt die Umformeinrichtung dafür, dass das transparente Element 22, dessen Fassung 24 oder ein anderes Element des Patienteninterfaces 6 umgeformt, beispielsweise eingeritzt oder eingeschnitten wird. Der Körper 54, 56 weist eine Härte auf, die größer als die Härte des transparenten Elements 22 und/oder der Fassung 24 ist. Er ritzt oder drückt in das transparente Element 22 und/oder die Fassung 24 eine Entwertungsmarkierung ein, insbesondere in eine Anti-Reflexions-Beschichtung.

Der Körper 54, 56 kann beispielsweise als Nadel, Dorn, Stachel, Pfeil oder Schneidkante ausgebildet sein und steht von der ophthalmologischen Vorrichtung 2, insbesondere von der Aufnahme 18, in Richtung des Patienteninterfaces 6 vor. Ist das Patienteninterface 6 über die Befestigungseinrichtung 52 an der Aufnahme 18 befestigt, besteht zwischen Körper 54 bzw. 56 und Patienteninterface 6 ein Spalt. Figur 2B zeigt analog zu Figur 1B eine Abwandlung der Bauweise der Figur 2A mit einem Kontaktglas 40 als transparentem Element.

Figur 2A/2B zeigt zwei Varianten in einer Zeichnung. In einer ersten Variante umfasst die Umformeinrichtung den Körper 54, der unbeweglich an der Aufnahme angebracht ist, und die Befestigungseinrichtung 52. Sie ist so ausgestaltet, dass sie beim Lösen des Patienteninterfaces 6 von der ophthalmologischen Vorrichtung 2 das Patienteninterface 6 auf die Aufnahme hinbewegt und so den Körper 54 zum Umformen in Kontakt mit dem Patienteninterface 6 bringt. Das Patienteninterface 6 ist dann, wie Figur 2A/2B zeigt, an der ophthalmologischen Vorrichtung 2 nicht mittels Unterdruck befestigt, sondern mithilfe der Befestigungseinrichtung 52 angebracht, die beispielsweise einen Bajonettverschluss umfasst. Das Patienteninterface 6 wird von der Befestigungseinrichtung 52 in Richtung des Körpers 54 bewegt, bevorzugt beim Lösen. Der Körper 54 dringt in das transparente Element 22 und/oder die Fassung 24 ein, sodass die Entwertungsmarkierung eingebracht wird. Die Befestigungseinrichtung 52 kann dabei ausgebildet sein, dass dabei eine Drehbewegung des Patienteninterfaces 6 erfolgt, so dass die Entwertungsmarkierung bogenförmig ist.

In einer zweiten Variante umfasst die Umformeinrichtung den Körper 56 und einen Antrieb 58 dafür, welcher von einem (in Figur 2A/2B nicht dargestellten) Steuergerät der ophthalmologischen Vorrichtung 2 angesteuert wird, und den Körper 56 umformend in das Patienteninterface 6 drückt. Dann bestehen an die Befestigung des Patienteninterfaces 6 an der Aufnahme 18 keine besonderen Anforderungen und es kann z. B. auch eine Vakuumbefestigung verwendet werden, die dann funktionell auch nicht der Umformeinrichtung zuzurechnen ist. Der Antrieb 58, welcher z. B. als Elektromotor ausgebildet sein kann, bewegt den Körper 56 derart auf das transparente Element 22 und/oder die Fassung 24 zu, dass die Strukturveränderung erzeugt wird. Vorzugsweise erfolgt dies, wenn der Benutzer der ophthalmologischen Vorrichtung 2 ein Signal gibt, das Patienteninterface 6 von der Aufnahme 18 zu lösen.

Die Strukturveränderung, welche von der Umformeinrichtung erzeugt wird, kann als Entwertungsmarkierung dienen. Sie wird dann vor einem Behandlungs- bzw. Untersuchungsbetrieb von der Vorrichtung 2 abgefragt, d. h. die Vorrichtung 2 prüft, ob eine Entwertungsmarkierung vorhanden ist. Ist dies der Fall, wird, wie bereits erläutert, der weitere Betrieb der Vorrichtung gesperrt.

In Ausführungsformen ist die Umformeinrichtung so ausgestaltet, dass sie eine Entwertungsmarkierung erzeugt, die bogenförmig ist. Hier ist die Detektion der Entwertungsmarkierung besonders einfach, da keine Gefahr besteht, dass der Körper 54 oder 56 die Entwertungsmarkierung vollständig überdeckt und damit nicht erkennbar macht. In anderen Ausführungsformen führt das Anbringen an der Aufnahme 18 eine rein axiale Bewegung aus. Dann ist eine laterale Belastung der Körper 54 oder 56 besonders gering.

In den Ausführungsformen mit feststehendem Körper 54 wird die Entwertungsmarkierung optional beim Anbringen des Patienteninterfaces 14 eingebracht. Bei der Überprüfung auf Wiederverwendung muss dann darauf geachtet werden, diese frische Entwertungsmarkierung von einer bereits eventuell vorhandenen, älteren Entwertungsmarkierung zu unterscheiden. Dies ist mit Mitteln der Bildverarbeitung aber jederzeit möglich, da die Wahrscheinlichkeit, dass eine neue Entwertungsmarkierung vollständig an derselben Stelle liegt, wie eine bereits bestehende Entwertungsmarkierung, vernachlässigbar gering ist.

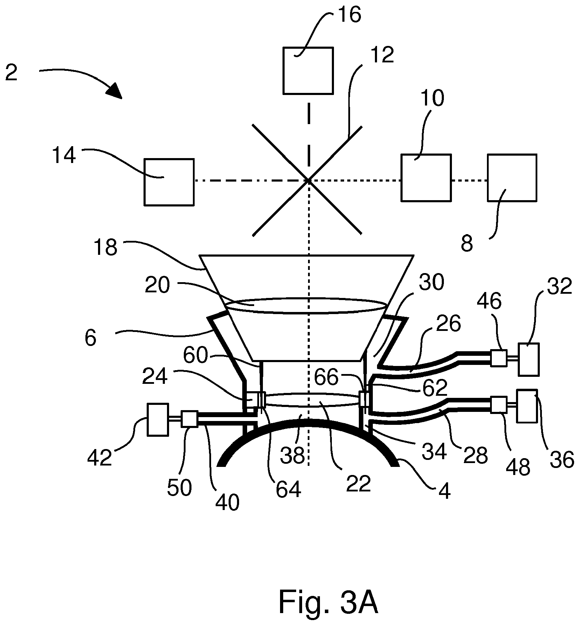

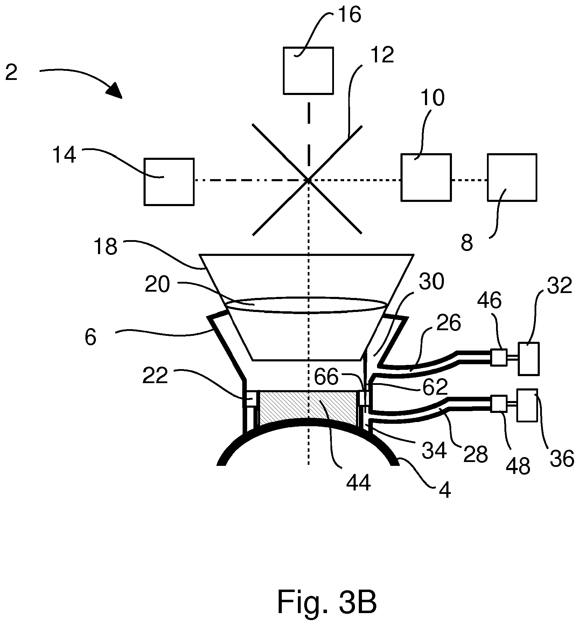

In den Figuren 3A und 3B dargestellten Ausführungsbeispielen weist die Umformeinrichtung eine Durchstoßeinrichtung 60 und/oder 62 auf, welche eine Nadel, einen Dorn, einen Stachel oder einen Pfeil umfasst. Die Durchstoßeinrichtung 60, 62 steht von der ophthalmologischen Vorrichtung 2, insbesondere von der Aufnahme 18, in Richtung des Patienteninterfaces 6 vor.

Figur 3A zeigt für die Umformeinrichtung zwei Ausführungsformen in einer Zeichnung. Eine erste Ausführungsform ist dadurch realisiert, dass eine Soll-Bruchstelle 64 in einer Trennwand zwischen Unterdruckkammer 30 und Flüssigkeitskammer 38 liegt. Diese Soll-Bruchstelle 64 wird von der Durchstoßeinrichtung 60 durchstoßen. In einer zweiten Ausführungsform, die in Figur 3A gezeigt ist, liegt die Soll-Bruchstelle 66 in einer Trennwand zwischen Unterdruckkammer 30 und Saugkanal 34. Sie wird von der Durchstoßeinrichtung 62 durchstoßen. In beiden Fällen ist erreicht, dass bei einer Wiederverwendung des Patienteninterfaces 6 die Unterdruckkammer 30 nicht mehr geschlossen werden kann, wenn das Patienteninterface 6 an die Aufnahme 18 angesetzt wird, da durch die Öffnung in der Soll-Bruchstelle 64 oder 66 eine Undichtigkeit besteht. Der Fall, dass eine Durchstoßeinrichtung zufällig eine bereits in der Soll-Bruchstelle bestehende Durchbohrung trifft und wieder ausreichend abdichtet, tritt praktisch nicht auf. In Ausführungsformen liegt die Soll-Bruchstelle 64, 66 im transparenten Element 22, durch welches die Strahlung der Laserquelle 8 geführt wird.

Für die Ausführungsformen mit Durchstoßeinrichtung gibt es zwei grundsätzliche Varianten. In einer ersten Variante durchdringt die Durchstoßeinrichtung 60, 62 die Soll-Bruchstelle 64, 66 nach Abschluss der Untersuchung/Behandlung, bevorzugt beim Lösen des Patienteninterfaces 6 von der Aufnahme 18. In einer zweiten Variante durchdringt die Durchstoßeinrichtung 60, 62 die Soll-Bruchstelle 64, 66 vor Abschluss der Untersuchung/Behandlung, bevorzugt beim Anbringen des Patienteninterfaces 6 an der Aufnahme 18. Dann ist die Soll-Bruchstelle 64 bzw. 66 als elastischer Durchstoßbereich ausgebildet, beispielsweise in Form einer Silikonmembran. Unter dem Begriff "elastisch" wird dabei eine Eigenschaft verstanden, die eine abdichtende Eigenschaft der Soll-Bruchstelle beschreibt. Hat die Durchstoßeinrichtung 60, 62 die Soll-Bruchstelle durchstoßen, tritt dennoch keine Undichtigkeit ein, solang die Durchstoßeinrichtung 60, 62 im Durchstoßbereich sitzt. Undichtigkeit ist erst gegeben, wenn die Durchstoßeinrichtung aus der Soll-Bruchstelle herausgezogen wird. Der Begriff "Soll-Bruchstelle" ist also im Zusammenhang mit dieser Ausführungsform darauf bezogen, dass er von der Durchstoßeinrichtung durchstoßen werden kann, sich dichtend an die Durchstoßeinrichtung anlegt, solange die Durchstoßeinrichtung im Durchstoßbereich sitzt, und nach der Entnahme der Durchstoßeinrichtung aus dem Durchstoßbereich eine Undichtigkeit hinsichtlich des entsprechenden Fluides (Gas oder Flüssigkeit) gegeben ist. Die Soll-Bruchstelle 64, 66 umschließt die Durchstoßeinrichtung 60, 62 fluiddicht, sodass die Unterdruckkammer 30 bzw. die Flüssigkeitskammer 38 fluiddicht sind, solange die Durchstoßeinrichtung in der jeweiligen Soll-Bruchstelle sitzt. Nach dem Lösen des Patienteninterfaces 6 weist die Soll-Bruchstelle ein Loch auf. Somit kann das Patienteninterface 6 nicht erneut angebracht werden.

Für die zum Durchstoßen nötige Relativbewegung zwischen Durchstoßeinrichtung und Patienteninterface 6 gilt das für die Ausführungsformen mit Körper 54, 56 zu Figuren 2A und 2B gesagte sinngemäß. Eine entsprechende Befestigungseinrichtung 52 oder ein Antrieb 58 sind möglich.

Wenn entsprechende Passungen für die Drehstellung, in der das Patienteninterface 6 befestigt wird, vorgesehen werden, genügt es, die Soll-Bruchstelle 64 bzw. 66 an derjenigen Stelle auszuführen, an welcher die Durchstoßeinrichtung 60 bzw. 62 auftreffen wird. Dies erleichtert den Fertigungsaufwand für das Patienteninterface 6. Ansonsten ist die Soll-Bruchstelle 64 bzw. 66 bogenförmig oder ringförmig ausgeführt.

Figur 3B zeigt eine Ausführungsform ähnlich der Figur 3A, wobei in Analogie zur Ausführungsform der Figuren 1B und 2B das transparente Element als am Auge anzulegendes Kontaktglas 44 ausgebildet ist. Der Durchstoßbereich 66 liegt hier dann in einer Wandung zwischen dem Saugkanal 34 und der Unterdruckkammer 30.

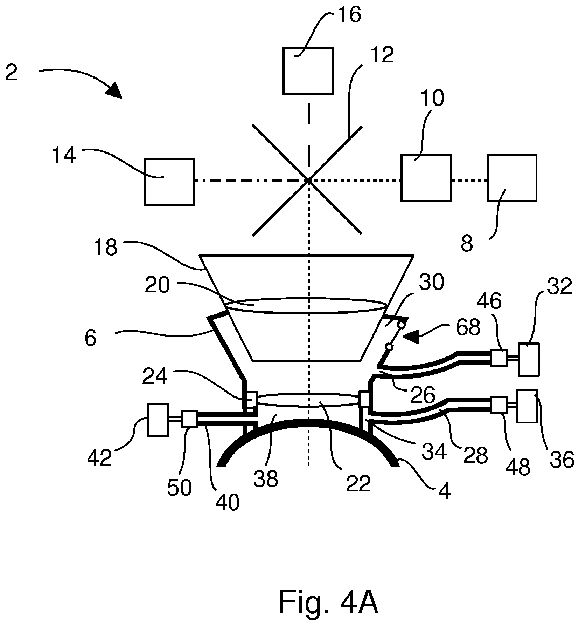

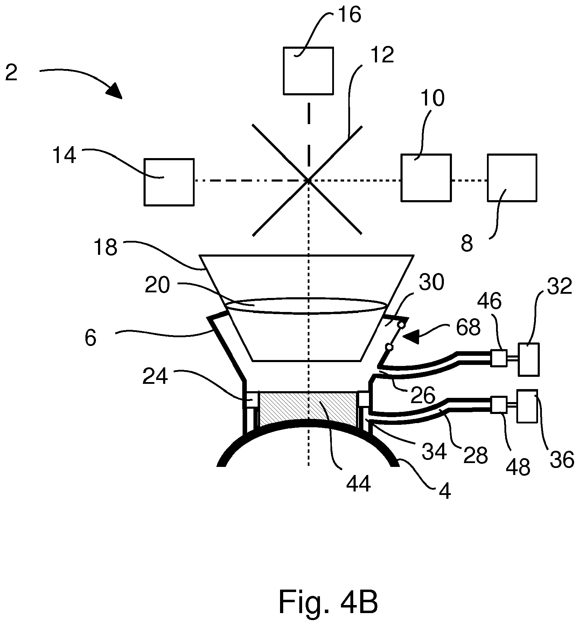

In den Ausführungsbeispielen der Figuren 4A und 4B, die sich wiederum hinsichtlich der Ausgestaltung des transparenten Elementes unterscheiden und einmal eine Flüssigkeitskammer 38 (Figur 4A) und ein anderes Mal ein Kontaktglas 44 (Figur 4B) vorsehen, weist eine Wandung einer Kammer des Patienteninterfaces 6, in der Unterdruck erzeugt wird, eine Soll-Bruchstelle in Form eines Berstfensters 68 auf. Es hält einer gewissen vorgegebenen Maximal-Druckdifferenz Stand, ohne zerstört zu werden. Die Maximal-Druckdifferenz ist größer als die in der Kammer vorgesehene. Beim Lösen des Patienteninterfaces 6 von der Aufnahme 18 wird in dieser Ausführungsform bewusst der Unterdruck in der Kammer kurzzeitig erhöht, und zwar so weit, bis das Berstfenster 68 bricht. Dann ist eine erneute Verwendung ausgeschlossen. Zugleich wird hierdurch die Kammer belüftet und das Patienteninterface 6 von der Aufnahme 18 gelöst. Figur 4A zeigt die Anordnung des Berstfensters 68 in einer Wandung, welche Teil der Unterdruckkammer 30 ist. Die Kammer ist somit die Unterdruckkammer 30. Diese Ausführungsform hat den Vorteil, dass die kurzzeitige Erhöhung des Unterdrucks derart, dass die Maximal-Druckdifferenz zur Umgebung überschritten wird, ausschließlich auf das Patienteninterface 6 und die Aufnahme 18, nicht jedoch auf den Patienten wirkt. Natürlich ist es aber auch gleichermaßen möglich, das Berstfenster 68 im Bereich des Saugkanals 34 vorzusehen. Die Kammer ist dann der vom Auge geschlossene Saugkanal 34. Ebenfalls ist es möglich, das Berstfenster 68 in einer Wandung zwischen Saugkanal 34 und Flüssigkeitskammer 38 oder in einer Wandung zwischen Unterdruckkammer 30 und Flüssigkeitskammer 38 anzuordnen.

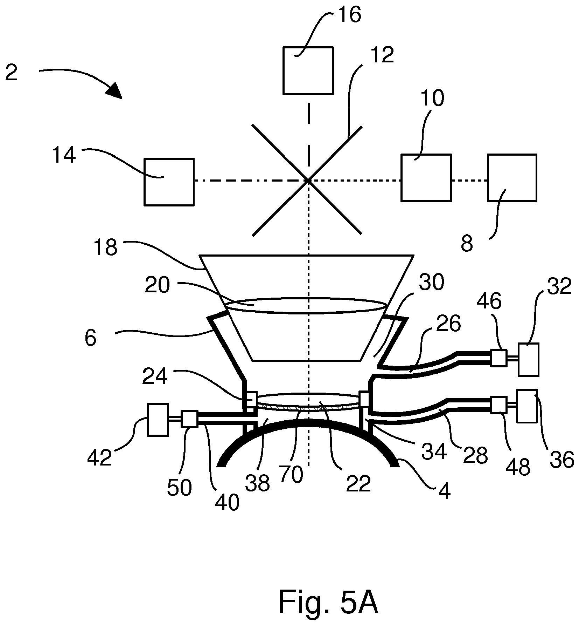

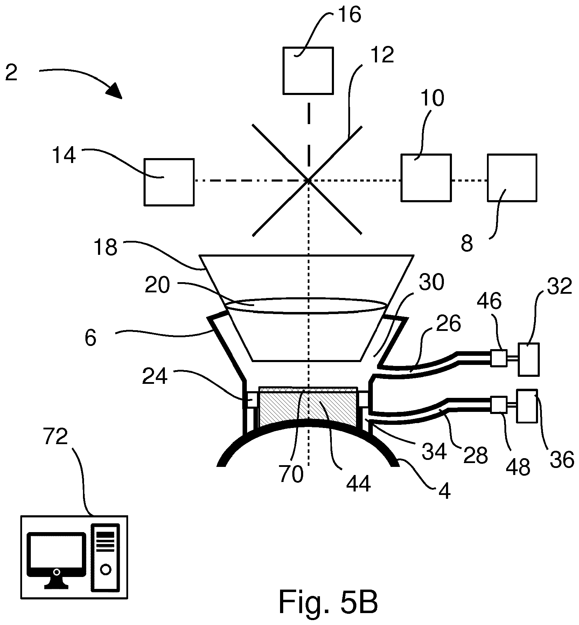

Das Patienteninterface 6 ist herstellerseitig sterilisiert. Bei einer Wiederverwendung müsste es erneut sterilisiert oder mindestens desinfiziert werden. Alternativ oder zusätzlich weist, wie die Figuren 5A und 5B zeigen, das Patienteninterface 6 eine reaktive Schicht 70 auf, welche im Falle einer Resterilisierung oder einer Desinfektion des Patienteninterfaces 6 eine bestimmte optische Eigenschaft annimmt, z. B. opak, intransparent und/oder farbig wird. Bei der herstellerseitigen Sterilisierung in der Herstellerverpackung und/oder mit ionisierender Strahlung bleibt die Schicht 70 hingegen transparent. Auf diese Weise ist mit bloßen Auge, aber optional durch eine Überprüfung mittels der ophthalmologischen Vorrichtung 2 unmittelbar erkennbar, ob das Patienteninterface 6 ein zweites Mal sterilisiert oder desinfiziert wurde. Somit kann festgestellt werden, ob das Patienteninterface 6 wiederverwendet wird, und in einem solchen Fall wird der weitere Betrieb der ophthalmologischen Vorrichtung 2 unterbunden. Die reaktive Schicht 70 ist an einer Oberfläche des transparenten Elements 30 vorgesehen. Sie kann beispielsweise eine transparente Polymerschicht aus Polycarbonat oder PMMA oder eine reaktive anorganische Beschichtung, z. B. aus Silber umfassen. Die reaktive Schicht 20 kann beispielsweise die optische Eigenschaft bei hohen Temperaturen (beispielsweise größer als 100° Celsius) und/oder hoher Luftfeuchtigkeit ändern, welche beim Autoklavieren oder Dampfsterilisieren auftreten. Ferner kann die reaktive Schicht 70 auf Wischdesinfektionsmittel Isopropanol, Glutaral oder Didecyldimethylammoniumchlorid, oder auf zur Gassterilisierung verwendete Substanzen, z. B. Ethylenoxid, reagieren.

In einer alternativen Ausführungsform kann man das Material, aus dem der Körper des Patienteninterfaces 6 gefertigt ist, derart auswählen, dass das Patienteninterface 6 sich bei den oben genannten Sterilisations- und Desinfektionsmethoden verformt, so dass es nicht mehr brauchbar ist, z. B. nicht mehr bündig an die Aufnahme 18 passt. In diesem Fall kann die Saugpumpe 32 nach erneuter Sterilisierung oder Desinfektion des Patienteninterfaces 6 keinen Unterdruck in der Kammer 30 aufbauen, da die mechanische Verformung zu einer Fluidundichtigkeit führt.

In der Ausführungsform gemäß Figur 5B ist das transparente Element wiederum als Kontaktglas 44 ausgeführt. In diesem Fall ist die reaktive Schicht 70 auf der Oberseite, die nicht im Kontakt mit dem Auge 4 steht, aufgebracht. Dies erlaubt eine größere Auswahl unter reaktiven Schichten 70, da auf Biokompatibilität nicht geachtet werden muss.

In Figur 5B ist ein Steuergerät 72 eingezeichnet. Es steuert den Betrieb der Vorrichtung 2, insbesondere beim Anbringen des Patienteninterfaces 6 an der Aufnahme 18. Das Steuergerät 72 ist Bestandteil der ophthalmologischen Vorrichtung 2 und ist optional auch in allen anderen Ausführungsformen, insbesondere den Ausführungsformen gemäß Figuren 1A bis 5A als Bestandteil der Vorrichtung 2 vorgesehen. Soweit in obiger Beschreibung Verfahrensschritte, beispielsweise eine Überprüfung auf Wiederverwendung, geschildert werden, steuert das Steuergerät 72 die ophthalmologische Vorrichtung 2 in einen entsprechenden Betrieb. Auch ist es die Steuereinrichtung 72, die bei einer erkannten Wiederverwendung einen weiteren Betrieb der Vorrichtung 2 blockiert.

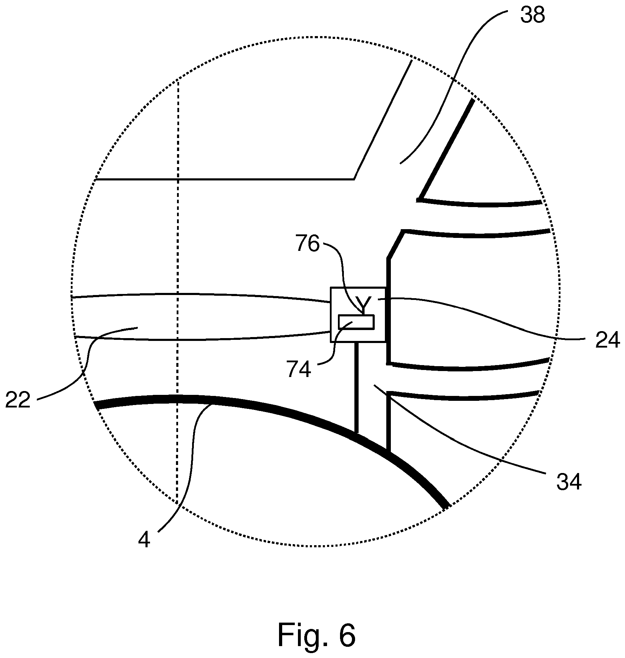

Figur 6 zeigt in einem vergrößerten Ausschnitt eine weitere Ausführungsform des Patienteninterfaces 6, die einen Funkchip 74 aufweist, der drahtlos Daten über das Patienteninterface 6 für die Vorrichtung 2 bereitstellt. Er hat dazu eine Antenne 76. Die Vorrichtung 2 weist eine entsprechende Lesevorrichtung für den Funkchip 74 auf. Dieser Funkchip 74 wird, um eine Wiederverwendung des Patienteninterfaces 6 zu verhindern, von der ophthalmologischen Vorrichtung 2 zerstört. Der Begriff "Zerstörung" ist bezüglich des Funkchips 74 auf dessen Funktionsfähigkeit bezogen. Es können also der Funkchip selbst oder damit verbundene Elemente, die für dessen Funktionieren nötig sind, zerstört werden. Beispiele für solche Elemente wären die Antenne 76 oder eine Antennenzuleitung. Die Zerstörung kann beispielsweise durch die im Zusammenhang mit anderen Ausführungsformen geschilderte Umformeinrichtung erfolgen. Die oben geschilderten Aspekte zur Erzeugung einer Relativbewegung zwischen Patienteninterface 6 und Körper/Durchstoßeinrichtung gelten gleichermaßen für die Ausführungsform mit dem Funkchip 74. Auch ist es möglich, dass die Laserstrahlung aus der Laserquelle 8 von der Laserstrahlführeinrichtung 10 bewusst auf den Funkchip 74 gerichtet wird, um diesen funktionsunfähig zu machen. Die Strukturveränderung sorgt dafür, dass mit dem Funkchip 74 keine Kommunikation mehr möglich ist. Die Vorrichtung 2 prüft deshalb bei einem Patienteninterface mit Funkchip 74, ob mit dem Funkchip 74 eine Kommunikation möglich ist. Schlägt eine Verbindungsaufnahme fehl, wird der weitere Betrieb der Vorrichtung 2 verhindert. Beim Funkchip kann es sich bevorzugt um einen RFID-Chip handeln. Hier ist es bevorzugt, dass die Leiterbahnen des Chips in ein Harz oder Polymer eingegossen sind oder in den Kunststoff, aus dem das Patienteninterface gefertigt ist. Diese Bauweise ist besonders zu bevorzugen, wenn der Funkchip mittels Laserstrahlung funktionsunfähig gemacht wird. Die Ursache besteht darin, dass beim Aufschmelzen von Leiterbahnen ansonsten Metall, z. B. Kupfer, in die Gasphase gelangen würde und sich möglicherweise auf Optiken der ophthalmologischen Vorrichtung absetzen könnte. Dadurch würden Reflexionseigenschaften der Optiken nachteilig verändert. Darüber hinaus könnten die Schleimhäute des Patienten in Kontakt mit gasförmigem Leiterbahnmaterial kommen, und es würde ein Geruch nach verbrannter Elektronik wahrnehmbar. Beides ist nicht gewünscht.

## Patentansprüche

1. Verfahren zum Sicherstellen der Sterilität eines Patienteninterfaces (6), das an einer ophthalmologischen Untersuchungs- und/oder Behandlungsvorrichtung (2) befestigt ist und zur Vakuumfixierung an einem Auge (4) eines Patienten ausgebildet ist, **dadurch gekennzeichnet, dass** mittels der ophthalmologischen Untersuchungs- und/oder Behandlungsvorrichtung (2) zur Verhinderung der Wiederverwendung eine Strukturveränderung am Patienteninterface (6) vorgenommen wird, die das Patienteninterface (6) hinsichtlich bestimmter optischer oder elektrischer oder Fluiddichtigkeitseigenschaften modifiziert.

2. Verfahren zum Sicherstellen der Sterilität eines Patienteninterfaces (6), das an einer ophthalmologischen Untersuchungs- und/oder Behandlungsvorrichtung (2) befestigt ist und zur Vakuumfixierung an einem Auge (4) eines Patienten ausgebildet ist, **dadurch gekennzeichnet, dass** vor Durchführung der Untersuchung und/oder Behandlung überprüft wird, ob das Patienteninterface (6) eine eine Wiederverwendung anzeigende Strukturveränderung aufweist, die das Patienteninterface (6) hinsichtlich bestimmter optischer oder elektrischer oder Fluiddichtigkeitseigenschaften modifiziert, und im positiven Fall ein Untersuchungs- und/oder Behandlungsbetrieb der ophthalmologischen Untersuchungs- und/oder Behandlungsvorrichtung (2) verhindert wird.

3. Verfahren nach Anspruch 1 oder 2, **dadurch gekennzeichnet, dass**
- die Strukturveränderung eine Veränderung von Reflexeigenschaften einer Beschichtung des Patienteninterfaces (6) umfasst, insbesondere einer Anti-Reflex-Beschichtung,
und/oder
- das Patienteninterface ein mit einer Fassung (24) versehenes transparentes Element (22) aufweist, durch welches Strahlung zum Auge (4) läuft, und die Strukturveränderung in der Fassung (24) oder das transparente Element (22) eingebracht wird,
und/oder
- die ophthalmologische Untersuchungs- und/oder Behandlungsvorrichtung (2) eine Laserquelle (8) zur Erzeugung eines Laserstrahls zur Behandlung des Auges (4) aufweist und die Strukturveränderung eine laserstrahlverursachte Markierung des Elements (22) oder der Fassung (24) umfasst, bevorzugt im Inneren des Elementes (2) bzw. der Fassung (24),
und/oder
- ein Daten bereitstellender Funkchip (74) am Patienteninterface (6) vorgesehen wird und die Strukturveränderung dessen elektrische Eigenschaften modifiziert, indem sie den Funkchip (74) funktionsunfähig macht, insbesondere durch Zerstörung einer Antenne (76) oder Antennenleitung.

4. Verfahren nach Anspruch 1, **dadurch gekennzeichnet, dass** die ophthalmologische Untersuchungs- und/oder Behandlungsvorrichtung (2) eine Umformeinrichtung (54, 56, 60, 62) zum mechanischen Umformen des Patienteninterfaces (6) aufweist und dass die Strukturveränderung eine Umformung des Patienteninterfaces (6) umfasst, insbesondere einen Ein- oder Durchstich, eine Ritzung oder einen Schnitt, wobei insbesondere die Umformeinrichtung einen Körper (54, 60, 62) zum Umformen des Patienteninterfaces (6), der an der ophthalmologischen Untersuchungs- und/oder Behandlungsvorrichtung (2) fest angeordnet ist und in Richtung auf das Patienteninterface (6) vorsteht, und eine Befestigungseinrichtung (52) zum Halten des Patienteninterfaces (6) an der ophthalmologischen Untersuchungs- und/oder Behandlungsvorrichtung (2) aufweist, wobei die Befestigungseinrichtung (52) beim Anbringen oder Lösen des Patienteninterfaces (6) dieses so auf den Körper (54, 60, 62) hin bewegt, dass dieser im Patienteninterface (6) die Strukturveränderung bewirkt, wobei bevorzugt die Befestigungseinrichtung (52) in Art eines Bajonettverschlusses ausgebildet ist, und/oder insbesondere die Umformeinrichtung einen Körper (56) zum Umformen des Patienteninterfaces (6) und einen Antrieb (58) zum Bewegen des Körpers (56) gegenüber dem Patienteninterface (6) aufweist und dass zum Ausbilden der Strukturveränderung der Körper (56) mittels des Antriebs (58) so auf das Patienteninterface (6) hin bewegt wird, dass der Körper (56) in das Patienteninterface (6) eindringt und es umformt.

5. Verfahren nach Anspruch 1, **dadurch gekennzeichnet, dass** die Strukturveränderung das Patienteninterface (6) hinsichtlich der Fluiddichtigkeitseigenschaften modifiziert, wobei die Strukturveränderung in einer Wandung einer Fluidkammer (30, 34, 38), ausgebildet ist, welche das Patienteninterface (6) zusammen mit der ophthalmologischen Untersuchungs- und/oder Behandlungsvorrichtung (2) und/oder dem Auge (4) bildet, wobei aufgrund der Strukturveränderung die Wandung mindestens nach dem Lösen des Patienteninterfaces (6) von der Untersuchungs- und/oder Behandlungsvorrichtung (2) nicht mehr fluiddicht ist, wobei insbesondere die Wandung Teil einer Vakuumkammer (30, 34) ist und eine Soll-Bruchstelle (68) aufweist, die bei einem vorgegebenen Differenzdruck bricht, wobei vor dem Lösen des Patienteninterfaces (6) ein Vakuum in der Vakuumkammer (30, 34) so erhöht wird, dass der vorgegebene Differenzdruck überschritten wird, so dass die Soll-Bruchstelle (68) bricht und dadurch die Strukturveränderung ausgebildet wird.

6. Verfahren nach Anspruch 1, **dadurch gekennzeichnet, dass** das Patienteninterface (6) mit einer Fluidleitung (26, 28, 40) mit einem Verbinder zur ophthalmologischen Untersuchungs- und/oder Behandlungsvorrichtung (2) bereitgestellt wird, wobei der Verbinder (46-50) als Einwegverbinder ausgestaltet ist, so dass eine Strukturveränderung die Veränderung des Einwegverbinders umfasst, die nach dem erstmaligen Lösen eintritt und kein erneutes fluiddichtes Anschließen mehr zulässt.

7. Verfahren zum Sicherstellen der Sterilität eines Patienteninterfaces (6), das an einer ophthalmologischen Untersuchungs- und/oder Behandlungsvorrichtung (2) eingesetzt wird,
- wobei das Patienteninterface (6) ein transparentes Element (22) aufweist, durch welches bei einem Betrieb der ophthalmologischen Untersuchungs- und/oder Behandlungsvorrichtung (2) Strahlung zum Auges (4) eines Patienten läuft, und eine reaktive Schicht (70) aufweist, die bei einer vorbestimmten Desinfektion des Patienteninterfaces (6) eine vorbestimmte optische Eigenschaft annimmt,
und/oder
- wobei das Patienteninterface (6) ein Strukturelement aus einem Material aufweist, welches bei Autoklaviertemperaturen erweicht, insbesondere oberhalb von 60, 80 oder 100 Grad Celsius.

8. Verfahren nach Anspruch 7, **dadurch gekennzeichnet, dass** vor Durchführung der Untersuchung und/oder Behandlung überprüft wird, ob das transparente Element (22) in einer Schicht (70) eine eine Wiederverwendung anzeigende, bestimmte optische Eigenschaft aufweist, welche die Schicht (70) bei einer vorbestimmten Sterilisierung oder einer Desinfektion des Patienteninterfaces (6) annimmt, und im positiven Fall ein Untersuchungs- und/oder Behandlungsbetrieb der ophthalmologischen Untersuchungs- und/oder Behandlungsvorrichtung (2) verhindert wird.

9. Ophthalmologische Untersuchungs- und/oder Behandlungsvorrichtung, die eine Aufnahme (18) zum Anbringen eines Patienteninterfaces (6) aufweist, das zum Verbinden eines Auges (4) eines Patienten mit der ophthalmologischen Untersuchungs- und/oder Behandlungsvorrichtung (2) dient, **dadurch gekennzeichnet, dass** die ophthalmologische Untersuchungs- und/oder Behandlungsvorrichtung (2) ausgebildet ist, zur Verhinderung der Wiederverwendung eine Strukturveränderung am Patienteninterface (6) vorzunehmen, die das Patienteninterface (6) hinsichtlich bestimmter optischer oder elektrischer oder Fluiddichtigkeitseigenschaften modifiziert.

10. Ophthalmologische Untersuchungs- und/oder Behandlungsvorrichtung nach Anspruch 9, **dadurch gekennzeichnet, dass** die ophthalmologische Untersuchungs- und/oder Behandlungsvorrichtung (2) ausgebildet ist, vor einem Untersuchungs- und/oder Behandlungsbetrieb zu überprüfen, ob das Patienteninterface (6) die Strukturveränderung aufweist und im positiven Fall einen Untersuchungs- und/oder Behandlungsbetrieb blockiert.

11. Ophthalmologische Untersuchungs- und/oder Behandlungsvorrichtung nach Anspruch 9 oder 10, **dadurch gekennzeichnet, dass**
- die Strukturveränderung eine Veränderung von Reflexeigenschaften einer Beschichtung des Patienteninterfaces (6) umfasst, insbesondere einer Anti-Reflex-Beschichtung,
und/oder
- die ophthalmologische Untersuchungs- und/oder Behandlungsvorrichtung (2) ausgebildet ist, die Strukturveränderung in eine Fassung (24) oder in ein von dieser gehaltenes transparentes Element (22) des Patienteninterfaces (6) einzubringen, und/oder
- die ophthalmologische Untersuchungs- und/oder Behandlungsvorrichtung (2) eine Laserquelle (8) zur Erzeugung eines Laserstrahls zur Behandlung des Auges (4) aufweist und die Strukturveränderung eine laserstrahlverursachte Markierung des Elements (22) oder der Fassung (24) umfasst, bevorzugt im Inneren des Elementes (2) bzw. der Fassung (24),
und/oder
- die Untersuchungs- und/oder Behandlungsvorrichtung ausgebildet ist, die Strukturveränderung in einer Wandung des Patienteninterfaces (6), die Teil einer Fluidkammer (30, 34, 38) auszubilden, welche das Patienteninterface (6) zusammen mit der ophthalmologischen Untersuchungs- und/oder Behandlungsvorrichtung (2) und/oder dem Auge (4) bildet, wobei aufgrund der Strukturveränderung die Wandung mindestens nach dem Lösen des Patienteninterfaces (6) von der Untersuchungs- und/oder Behandlungsvorrichtung (2) nicht mehr fluiddicht ist,
und/oder
- die Untersuchungs- und/oder Behandlungsvorrichtung (2) eine Vakuumquelle (32, 36) aufweist und ausgebildet ist, vor dem Lösen des Patienteninterfaces (6) ein Vakuum so zu erhöhen, dass ein vorgegebener Unterdruck überschritten wird, so dass eine Soll-Bruchstelle (68) in einer Wandung des Patienteninterfaces (6) bricht und dadurch die Strukturveränderung ausgebildet ist,
und/oder
- die Strukturveränderung elektrische Eigenschaften eines Daten bereitstellenden Funkchips (74) am Patienteninterface (6) modifiziert, indem sie den Funkchip (74) funktionsunfähig macht, was insbesondere eine Zerstörung einer Antenne (76) oder einer Antennenleitung umfasst, wobei insbesondere die ophthalmologische Untersuchungs- und/oder Behandlungsvorrichtung (2) eine Laserquelle (8) zur Erzeugung eines Laserstrahls zur Behandlung des Auges (4) aufweist und ausgebildet ist, den Laserstrahl auf den Funkchip (74) oder damit verbundene elektrische Elemente, insbesondere eine Funkantenne oder Funkantennenleitung, zu richten, um die Strukturveränderung auszubilden.

12. Ophthalmologische Untersuchungs- und/oder Behandlungsvorrichtung nach einem der Ansprüche 9 - 11,
**dadurch gekennzeichnet, dass** die ophthalmologische Untersuchungs- und/oder Behandlungsvorrichtung (2) eine Umformeinrichtung aufweist, die einen Körper (54, 56, 60, 62) zum Umformen des Patienteninterfaces (6) aufweist und ausgebildet ist, die Strukturveränderung durch Umformung des Patienteninterfaces (6) vorzunehmen, insbesondere durch einen Ein- oder Durchstich, eine Ritzung oder einen Schnitt, wobei insbesondere der Körper (54, 60, 62) zum Umformen des Patienteninterfaces (6) an der ophthalmologischen Untersuchungs- und/oder Behandlungsvorrichtung (2) fest angeordnet ist und in Richtung auf das Patienteninterface (6) vorsteht und dass die Umformeinrichtung eine Befestigungseinrichtung (52) zum Befestigen des Patienteninterfaces an der ophthalmologischen Untersuchungs- und/oder Behandlungsvorrichtung (2) aufweist, die ausgebildet ist, beim Anbringen oder Lösen des Patienteninterfaces (6) dieses so auf den Körper (54, 60, 62) hin zu bewegen, dass dieser im Patienteninterface (6) die Strukturveränderung durch Umformung bewirkt, wobei bevorzugt die Befestigungseinrichtung (52) in Art eines Bajonettverschlusses ausgebildet ist, oder wobei insbesondere die Umformeinrichtung einen Antrieb (58) zum Bewegen des Körpers (56) gegenüber dem Patienteninterface (6) aufweist und ausgebildet ist, den Körper (56) so auf das Patienteninterface (6) hin zu bewegen, dass der Körper (56) in das Patienteninterface (6) eindringt und es umformt.

13. Ophthalmologische Untersuchungs- und/oder Behandlungsvorrichtung, die eine Aufnahme (18) zum Anbringen eines Patienteninterfaces (6) aufweist, das zum Verbinden eines Auges (4) eines Patienten mit der ophthalmologischen Untersuchungs- und/oder Behandlungsvorrichtung (2) dient und ein transparentes Element (22), durch welches Strahlung zum Auge (4) läuft, aufweist, **dadurch gekennzeichnet,**
**dass** die ophthalmologische Untersuchungs- und/oder Behandlungsvorrichtung (2) vor einem Untersuchungs- und/oder Behandlungsbetrieb überprüft, ob das transparente Element (22) in einer Schicht (70) eine eine Wiederverwendung anzeigende, vorbestimmte optische Eigenschaft aufweist, welche die Schicht (70) bei einer vorbestimmten Sterilisierung oder einer Desinfektion des Patienteninterfaces (6) ändert, und im positiven Fall einen Untersuchungs- und/oder Behandlungsbetrieb blockiert.

14. Patienteninterface zum Verbinden eines Auges (4) eines Patienten mit einer ophthalmologischen Untersuchungs- und/oder Behandlungsvorrichtung (2), das zur Unterdruckbefestigung am Auge (4) und/oder der Untersuchungs- und/oder Behandlungsvorrichtung (2) ausgebildet ist und mindestens eine Fluidleitung (26, 28, 40) zur Befestigung an einem Anschluss der ophthalmologischen Untersuchungs- und/oder Behandlungsvorrichtung (2) aufweist, **dadurch gekennzeichnet, dass** die Fluidleitung (26, 28, 40) einen Verbinder (46-50) aufweist, der als Einwegverbinder ausgestaltet ist, so dass nach dem erstmaligen Lösen des Einwegverbinders kein erneutes fluiddichtes Anschließen mehr möglich ist, oder dass das Patienteninterface (6) eine zur Unterdruckbefestigung benötigte Wandung und darin eine Soll-Bruchstelle (68) aufweist, die bei einem vorgegebenen Differenzdruck bricht.

15. Patienteninterface zum Verbinden eines Auges (4) eines Patienten mit einer ophthalmologischen Untersuchungs- und/oder Behandlungsvorrichtung (2), umfassend ein transparentes Element (22), wobei
- das Patienteninterface (6) ein transparentes Element (22) aufweist durch welches Strahlung zum Auge (4) eines Patienten führbar ist und das eine reaktive Schicht (70) aufweist, die
bei einer vorbestimmten Sterilisierung oder einer Desinfektion des Patienteninterfaces (6) eine vorbestimmte optische Eigenschaft annimmt, wobei insbesondere die reaktive Schicht (70) die vorbestimmte optische Eigenschaft oberhalb einer vorgegebenen Temperatur und/oder bei Kontakt mit einem Desinfektionsfluid und/oder bei einer zweiten Sterilisierung mit ionisierender Strahlung annimmt,
und/oder
- das Strukturelement ein Material aufweist, welches bei Autoklaviertemperaturen erweicht, insbesondere oberhalb von 60, 80 oder 100 Grad Celsius.

## Claims

1. Method for ensuring the sterility of a patient interface (6) which is fastened to an ophthalmological examination and/or treatment apparatus (2) and which is embodied to be fixed to an eye (4) of a patient by vacuum, **characterized in that** the ophthalmological examination and/or treatment apparatus (2) makes a change in the structure on the patient interface (6) for the purposes of preventing reuse, said structure change modifying the patient interface (6) in respect of certain optical or electrical or fluid-tight properties.

2. Method for ensuring the sterility of a patient interface (6) which is fastened to an ophthalmological examination and/or treatment apparatus (2) and which is embodied to be fixed to an eye (4) of a patient by vacuum, **characterized in that** a check is carried out before the examination and/or treatment as to whether the patient interface (6) has a structure change that indicates reuse, said structure change modifying the patient interface (6) in respect of certain optical or electrical or fluid-tight properties, and, if so, an examination and/or treatment operation of the ophthalmological examination and/or treatment apparatus (2) is prevented.

3. Method according to Claim 1 or 2, **characterized in that**
- the structure change comprises a change in reflection properties of a coating of the patient interface (6), in particular of an antireflection coating,
and/or
- the patient interface comprises a transparent element (22) provided with a mount (24), radiation passing to the eye (4) through said element, and the structure change is introduced into the mount (24) or the transparent element (22),
and/or
- the ophthalmological examination and/or treatment apparatus (2) comprises a laser source (8) for generating a laser beam for treating the eye (4) and the structure change comprises a laser beam-induced marking of the element (22) or the mount (24), preferably in the interior of the element (2) or the mount (24),
and/or
- a data-providing radio chip (74) is provided at the patient interface (6) and the structure change modifies the electrical properties of said radio chip by virtue of rendering the radio chip (74) inoperative, in particular by destroying an antenna (76) or antenna line.

4. Method according to Claim 1, **characterized in that** the ophthalmological examination and/or treatment apparatus (2) comprises a reshaping device (54, 56, 60, 62) for mechanically reshaping the patient interface (6) and **in that** the structure change comprises the patient interface (6) being reshaped, in particular by way of a puncture or breakthrough, scarring or cut, wherein in particular the reshaping device comprises a body (54, 60, 62) for reshaping the patient interface (6), said body being securely arranged at the ophthalmological examination and/or treatment apparatus (2) and protruding in the direction of the patient interface (6), and a fastening device (52) for holding the patient interface (6) against the ophthalmological examination and/or treatment apparatus (2), wherein the fastening device (52) moves the patient interface (6) to the body (54, 60, 62) when attaching or detaching said patient interface in such a way that the body causes the structure change in the patient interface (6), wherein the fastening device (52) is preferably embodied in the style of a bayonet closure, and/or in particular the reshaping device comprises a body (56) for reshaping the patient interface (6) and a drive (58) for moving the body (56) in relation to the patient interface (6) and that the body (56) is moved towards the patient interface (6) by means of the drive (58) for the purposes of forming the structure change, in such a way that the body (56) penetrates into the patient interface (6) and reshapes the latter.

5. Method according to Claim 1, **characterized in that** the structure change modifies the patient interface (6) in respect of the fluid-tight properties, wherein the structure change is embodied in a wall of a fluid chamber (30, 34, 38) which forms the patient interface (6) together with the ophthalmological examination and/or treatment apparatus (2) and/or the eye (4), wherein the wall is no longer fluid tight on account of the structure change, at least once the patient interface (6) has been detached from the examination and/or treatment apparatus (2), wherein in particular the wall is part of the vacuum chamber (30, 34) and has a predetermined breaking point (68) which breaks at a specified differential pressure, wherein a vacuum in the vacuum chamber (30, 34) is increased before the patient interface (6) is detached so that the specified differential pressure is exceeded and so the predetermined breaking point (68) breaks and thereby forms the structure change.

6. Method according to Claim 1, **characterized in that** the patient interface (6) is provided with a fluid conduit (26, 28, 40) with a connector to the ophthalmological examination and/or treatment apparatus (2), wherein the connector (46-50) is configured as a disposable connector such that a structure change comprises the alteration of the disposable connector which occurs after the first detaching process and which no longer allows renewed fluid-tight sealing.

7. Method for ensuring the sterility of a patient interface (6) which is used on an ophthalmological examination and/or treatment apparatus (2),
- wherein the patient interface (6) comprises a transparent element (22), through which radiation passes to the eye (4) of a patient during operation of the ophthalmological examination and/or treatment apparatus (2), and comprises a reactive layer (70) which adopts a predetermined optical property in the case of a predetermined disinfection of the patient interface (6)
and/or
- wherein the patient interface (6) comprises a structure element made of a material that softens at autoclaving temperatures, in particular above 60, 80 or 100 degrees Celsius.

8. Method according to Claim 7, **characterized in that** a check is carried out before the examination and/or treatment as to whether the transparent element (22) has a certain optical property that indicates reuse in a layer (70), the layer (70) adopting said optical property in the case of a predetermined sterilization or disinfection of the patient interface (6), and, if so, an examination and/or treatment operation of the ophthalmological examination and/or treatment apparatus (2) is prevented.

9. Ophthalmological examination and/or treatment apparatus which comprises a receptacle (18) for attaching a patient interface (6) that serves to connect an eye (4) of a patient to the ophthalmological examination and/or treatment apparatus (2), **characterized in that** the ophthalmological examination and/or treatment apparatus (2) is embodied to make a change in the structure on the patient interface (6) for the purposes of preventing reuse, said structure change modifying the patient interface (6) in respect of certain optical or electrical or fluid-tight properties.

10. Ophthalmological examination and/or treatment apparatus according to Claim 9, **characterized in that** the ophthalmological examination and/or treatment apparatus (2) is embodied to carry out a check before an examination and/or treatment operation as to whether the patient interface (6) has a structure change and, if so, block an examination and/or treatment operation.

11. Ophthalmological examination and/or treatment apparatus according to Claim 9 or 10, **characterized in that**
- the structure change comprises a change in reflection properties of a coating of the patient interface (6), in particular of an antireflection coating,
and/or
- the ophthalmological examination and/or treatment apparatus (2) is embodied to introduce the structure change into a mount (24) or into a transparent element (22) of the patient interface (6) held by said mount, and/or
- the ophthalmological examination and/or treatment apparatus (2) comprises a laser source (8) for generating a laser beam for treating the eye (4) and the structure change comprises a laser beam-induced marking of the element (22) or the mount (24), preferably in the interior of the element (2) or the mount (24),
and/or
- the examination and/or treatment apparatus is embodied to form the structure change in a wall of the patient interface (6) which is part of a fluid chamber (30, 34, 38) which forms the patient interface (6) together with the ophthalmological examination and/or treatment apparatus (2) and/or the eye (4), wherein the wall is no longer fluid tight on account of the structure change, at least once the patient interface (6) has been detached from the examination and/or treatment apparatus (2),
and/or
- the examination and/or treatment apparatus (2) comprises a vacuum source (32, 36) and is embodied to increase the vacuum before the patient interface (6) is detached so that a specified negative pressure is exceeded and so that a predetermined breaking point (68) in a wall of the patient interface (6) breaks and thereby forms the structure change,
and/or
- the structure change modifies electrical properties of a data-providing radio chip (74) at the patient interface (6) by virtue of rendering the radio chip (74) inoperative, this comprising in particular a destruction of an antenna (76) or an antenna line, wherein in particular the ophthalmological examination and/or treatment apparatus (2) comprises a laser source (8) for generating a laser beam for treating the eye (4) and is embodied to direct the laser beam at the radio chip (74) or electrical elements connected therewith, in particular a radio antenna or a radio antenna line, in order to form the structure change.

12. Ophthalmological examination and/or treatment apparatus according to any one of Claims 9-11, **characterized in that** the ophthalmological examination and/or treatment apparatus (2) comprises a reshaping device which comprises a body (54, 56, 60, 62) for reshaping the patient interface (6) and is embodied to make the structure change by reshaping the patient interface (6), in particular by a puncture or breakthrough, scarring or cut, wherein in particular the body (54, 60, 62) for reshaping the patient interface (6) is fixedly arranged at the ophthalmological examination and/or treatment apparatus (2) and protrudes in the direction of the patient interface (6), and **in that** the reshaping device comprises a fastening device (52) for fastening the patient interface to the ophthalmological examination and/or treatment apparatus (2) which is embodied to move this to the body (54, 60, 62) when attaching or detaching the patient interface (6), in such a way that said body causes the structure change in the patient interface (6) by reshaping, wherein the fastening device (52) is preferably embodied in the style of a bayonet closure, or wherein in particular the reshaping device comprises a drive (58) for moving the body (56) in relation to the patient interface (6) and is embodied to move the body (56) to the patient interface (6) in such a way that the body (56) penetrates into the patient interface (6) and reshapes the latter.

13. Ophthalmological examination and/or treatment apparatus which comprises a receptacle (18) for attaching a patient interface (6) that serves to connect an eye (4) of a patient to the ophthalmological examination and/or treatment apparatus (2), and a transparent element (22) through which radiation passes to the eye (4), **characterized in that** before an examination and/or treatment operation the ophthalmological examination and/or treatment apparatus (2) checks whether the transparent element (22) has a predetermined optical property that indicates reuse in a layer (70), said optical property changing the layer (70) in the case of a predetermined sterilization or disinfection of the patient interface (6), and, if so, blocks an examination and/or treatment operation.

14. Patient interface for connecting an eye (4) of a patient to an ophthalmological examination and/or treatment apparatus (2), embodied to be fastened to the eye (4) and/or the examination and/or treatment apparatus (2) by way of negative pressure and comprising at least one fluid conduit (26, 28, 40) serving to be fastened to a connector of the ophthalmological examination and/or treatment apparatus (2), **characterized in that** the fluid conduit (26, 28, 40) comprises a connector (46-50) which is configured as a disposable connector such that after the first detaching process of the disposable connector a renewed fluid-tight connection is no longer possible, or **in that** the patient interface (6) comprises a wall required for negative pressure fastening and a predetermined breaking point (68) therein, the latter breaking at a specified differential pressure.

15. Patient interface for connecting an eye (4) of a patient to an ophthalmological examination and/or treatment apparatus (2), comprising a transparent element (22), wherein
- the patient interface (6) comprises a transparent element (22), through which it is possible to pass radiation to the eye (4) of a patient and which comprises a reactive layer (70) that adopts a predetermined optical property in the case of a predetermined sterilization or a disinfection of the patient interface (6), wherein in particular the reactive layer (70) adopts the predetermined optical property above a specified temperature and/or upon contact with a disinfection fluid and/or during a second sterilization with ionising radiation,
and/or
- the structure element comprises a material that softens at autoclaving temperatures, in particular above 60, 80 or 100 degrees Celsius.

## Revendications

1. Procédé destiné à assurer la stérilité d'une interface patient (6) qui est fixée à un dispositif d'examen et/ou de traitement ophtalmologique (2) et qui est conçue pour être fixée sous vide à un œil (4) d'un patient, **caractérisé en ce qu'**une modification structurelle est apportée à l'interface patient (6), laquelle modifie l'interface patient (6) quant à des propriétés optiques, électriques ou d'étanchéité aux fluides, au moyen du dispositif d'examen et/ou de traitement ophtalmologique (2) afin éviter sa réutilisation.

2. Procédé destiné à assurer la stérilité d'une interface patient (6) qui est fixée à un dispositif d'examen et/ou de traitement ophtalmologique (2) et qui est destinée à être fixée sous vide à un œil (4) d'un patient, **caractérisé en ce qu'**avant d'effectuer l'examen et/ou le traitement, il est vérifié si l'interface patient (6) présente une modification structurelle, indiquant une réutilisation, qui modifie l'interface patient (6) quant à des propriétés optiques, électriques ou d'étanchéité aux fluides, et, si c'est le cas, une opération d'examen et/ou de traitement du dispositif d'examen et/ou de traitement ophtalmologique (2) est empêchée.

3. Procédé selon la revendication 1 ou 2, **caractérisé en ce que**
- la modification structurelle comprend une modification de propriétés réfléchissantes d'un revêtement de l'interface patient (6), en particulier d'un revêtement antireflet,
et/ou
- l'interface patient comporte un élément transparent (22), pourvu d'une monture (24}, à travers lequel le rayonnement se propage vers l'œil (4) et la modification structurelle est introduite dans la monture (24) ou l'élément transparent (22),
et/ou
- le dispositif d'examen et/ou de traitement ophtalmologique (2) comporte une source laser (8) destinée à générer un faisceau laser pour traiter l'œil (4) et la modification structurelle comprend un marquage induit par faisceau laser de l'élément (22) ou de la monture (24), de préférence à l'intérieur de l'élément (2) ou de la monture (24),
et/ou
- une puce radio (74) fournissant des données est prévue au niveau de l'interface patient (6) et la modification structurelle modifie ses propriétés électriques **en ce que** la puce radio (74) est rendue inopérante, en particulier **en ce qu'**une antenne (76) ou une ligne d'antenne est détruite.

4. Procédé selon la revendication 1, **caractérisé en ce que** le dispositif d'examen et/ou de traitement ophtalmologique (2) comporte un mécanisme de déformation (54, 56, 60, 62) destiné à déformer mécaniquement l'interface patient (6) et **en ce que** la modification structurelle comprend une déformation de l'interface patient (6), notamment une piqûre traversante ou non, une entaille ou une coupure, en particulier le mécanisme de déformation comportant un corps (54, 60, 62) destiné à déformer l'interface patient (6), qui est disposé de manière fixe sur le dispositif d'examen et/ou de traitement ophtalmologique (2) et qui fait saillie en direction de l'interface patient (6), et un mécanisme de fixation (52) destiné à maintenir l'interface patient (6) sur le dispositif d'examen et/ou de traitement ophtalmologique (2), le mécanisme de fixation (52) déplaçant l'interface patient (6), lors du montage ou du démontage de celui-ci, vers le corps (54, 60, 62) de telle manière que celui-ci provoque la modification structurelle de l'interface patient (6), le mécanisme de fixation (52) étant de préférence conçu sous la forme d'une fermeture à baïonnette, et/ou en particulier le mécanisme de déformation comportant un corps (56) destiné à déformer l'interface patient (6) et un entraînement (58) destiné à déplacer le corps (56) par rapport à l'interface patient (6) et **en ce que**, pour effectuer la modification structurelle, le corps (56) est déplacé vers l'interface patient (6) au moyen de l'entraînement (58) de telle manière que le corps (56) pénètre dans l'interface patient (6) et la déforme.

5. Procédé selon la revendication 1, **caractérisé en ce que** la modification structurelle modifie l'interface patient (6) quant aux propriétés d'étanchéité aux fluides, la modification structurelle étant effectuée dans une paroi d'une chambre à fluide {30, 34.38) qui forme l'interface patient (6) conjointement avec le dispositif d'examen et/ou de traitement ophtalmologique (2) et/ou l'œil (4), en raison de la modification structurelle, la paroi n'étant plus étanche aux fluides au moins après le démontage de l'interface patient {6} du dispositif d'examen et/ou de traitement (2), en particulier la paroi faisant partie d'une chambre à vide (30, 34) et comportant un point de rupture (68) qui se rompt à une pression différentielle spécifiée, avant de démonter l'interface patient (6), un vide dans la chambre à vide (30.34) étant augmenté de façon à devenir supérieur à la pression différentielle spécifiée de sorte que le point de rupture (68) se brise et que la modification structurelle s'effectue.

6. Procédé selon la revendication 1, **caractérisé en ce que** l'interface patient (6) est fournie avec une conduite de fluide (26, 28,40) pourvue d'un connecteur destiné au dispositif d'examen et/ou de traitement ophtalmologique (2), le connecteur (46-50) étant conçu comme un connecteur unidirectionnel de sorte qu'une modification structurelle comprend la modification du connecteur unidirectionnel qui se produit après le premier démontage et qui ne permet plus un nouveau raccordement étanche aux fluides.

7. Procédé destiné à assurer la stérilité d'une interface patient (6) qui est utilisée sur un dispositif d'examen et/ou de traitement ophtalmologique (2),
- l'interface patient (6) comportant un élément transparent (22) à travers lequel le rayonnement se propage vers l'œil (4) d'un patient lorsque le dispositif d'examen et/ou de traitement ophtalmique (2) est en fonctionnement, et comportant une couche réactive (70) qui présente une propriété optique prédéterminée lors d'une désinfection prédéterminée de l'interface patient (6),
et/ou
- l'interface patient (6) comportant un élément structurel en un matériau qui se ramollit à des températures d'autoclavage, en particulier au-dessus de 60, 80 ou 100 degrés Celsius.

8. Procédé selon la revendication 7, **caractérisé en ce que**, avant d'effectuer l'examen et/ou le traitement, il est vérifié si l'élément transparent (22) présente dans une couche (70) une propriété optique déterminée qui indique une réutilisation et qui se charge de la couche (70) lors d'une stérilisation prédéterminée ou d'une désinfection de l'interface patient (6), et, si c'est le cas, une opération d'examen et/ ou de traitement du dispositif d'examen et/ou de traitement ophtalmologique (2) est empêchée.

9. Dispositif d'examen et/ou de traitement ophtalmologique qui comporte un réceptacle (18) destiné au montage d'une interface patient (6) qui sert à relier un œil (4) d'un patient au dispositif d'examen et/ou de traitement ophtalmologique (2), **caractérisé en ce que** le dispositif d'examen et/ou de traitement ophtalmologique (2) est conçu pour effectuer une modification structurelle au niveau de l'interface patient (6), qui modifie l'interface patient (6) quant à des propriétés optiques ou électriques ou d'étanchéité aux fluides, afin d'empêcher la réutilisation.

10. Dispositif d'examen et/ou de traitement ophtalmologique selon la revendication 9, **caractérisé en ce que** le dispositif d'examen et/ou de traitement ophtalmologique (2) est adapté pour vérifier avant une opération d'examen et/ou de traitement si l'interface patient (6) présente la modification structurelle et, si c'est le cas, bloque une opération d'examen et/ou de traitement.

11. Dispositif d'examen et/ou de traitement ophtalmologique selon la revendication 9 ou 10, **caractérisé en ce que**
- la modification structurelle comprend une modification de propriétés réfléchissantes d'un revêtement de l'interface patient (6), en particulier d'un revêtement antireflet, et/ou
- le dispositif d'examen et/ou de traitement ophtalmologique (2) est conçu pour introduire la modification structurelle dans une monture (24) ou dans un élément transparent (22) de l'interface patient (6) qui est maintenu par ladite monture, et/ou
- le dispositif d'examen et/ou de traitement ophtalmologique (2) comporte une source laser (8) destinée à générer un faisceau laser pour le traitement de l'œil (4) et la modification structurelle comprend le marquage de l'élément (22) ou de la monture (24) provoqué par faisceau laser, de préférence à l'intérieur de l'élément (2) ou de la monture (24), et/ou
- le dispositif d'examen et/ou de traitement est conçu pour effectuer la modification structurelle dans une paroi de l'interface patient (6) qui fait la partie d'une chambre à fluide (30, 34, 38) que l'interface patient (6) forme conjointement avec le dispositif d'examen et/ou de traitement ophtalmologique (2) et/ou l'œil (4), en raison de la modification structurelle la paroi n'est plus étanche aux fluides au moins après le démontage de l'interface patient (6) du dispositif d'examen et/ou de traitement (2),
et/ou
- le dispositif d'examen et/ou de traitement (2) comporte une source de vide (32, 36) et est conçu pour augmenter un vide avant le démontage de l'interface patient (6) de manière à ce qu'il devienne supérieur à une dépression spécifiée de sorte qu'un point de rupture (68) se brise dans une paroi de l'interface patient (6) et qu'ainsi la modification structurelle s'effectue,
et/ou
- la modification structurelle modifie des propriétés électriques d'une puce radio (74) fournissant des données au niveau de l'interface patient (6) **en ce que** la puce radio (74) est rendue inopérante, ce qui comprend notamment la destruction d'une antenne (76) ou d'une ligne d'antenne, notamment le dispositif d'examen et/ou de traitement ophtalmologique (2) comportant une source laser (8) destinée à générer un faisceau laser pour le traitement de l'œil (4) et étant conçu pour diriger le faisceau laser sur la puce radio (74) ou des éléments électriques qui sont reliés à celle-ci, notamment une antenne radio ou une ligne d'antenne radio afin d'effectuer la modification structurelle.

12. Dispositif d'examen et/ou de traitement ophtalmologique selon l'une des revendications 9 à 11,
**caractérisé en ce que** le dispositif d'examen et/ou de traitement ophtalmologique (2) comporte un mécanisme de déformation qui comporte un corps (54, 56, 60, 62) destiné à déformer l'interface patient (6) et qui est conçu pour effectuer la modification structurelle par déformation de l'interface patient (6), notamment par une piqûre traversante ou non, une entaille ou une coupure, notamment le corps (54, 60, 62) de déformation de l'interface patient (6) étant disposé de manière fixe au niveau du dispositif d'examen et/ou de traitement ophtalmologique (2) et faisant saillie en direction de l'interface patient (6) et **en ce que** le mécanisme de déformation comporte un mécanisme de fixation (52) qui est destiné à fixer l'interface patient au dispositif d'examen et/ou de traitement ophtalmologique (2) et qui est conçu pour déplacer l'interface patient (6), lors du montage ou du démontage de celui-ci, vers le corps (54, 60, 62) de sorte que celui-ci provoque dans l'interface patient (6) une modification structurelle, le dispositif de fixation (52) étant de préférence conçu comme une fermeture à baïonnette, ou en particulier le mécanisme de déformation comporte un entraînement (58) destiné à déplacer le corps (56) par rapport à l'interface patient (6) et étant conçu pour déplacer le corps (56) vers l'interface patient (6) de telle manière que le corps (56) pénètre dans l'interface patient (6) et la déforme.

13. Dispositif d'examen et/ou de traitement ophtalmologique qui comporte un réceptacle (18} destiné à monter une interface patient (6} qui sert à relier un œil (4) d'un patient au dispositif d'examen et/ou de traitement ophtalmologique (2) et qui comporte un élément transparent (22) à travers lequel le rayonnement se propage vers l'œil (4), **caractérisé en ce que**
le dispositif d'examen et/ou de traitement ophtalmologique (2} vérifie, avant une opération d'examen et/ou de traitement, si l'élément transparent (22) présente dans une couche (70) une propriété optique prédéterminée qui indique une réutilisation et qui modifie la couche (70) lors d'une stérilisation prédéterminée ou d'une désinfection de l'interface patient (6) et, si c'est le cas, bloque une opération d'examen et/ou de traitement.

14. Interface patient qui est destinée à relier un œil (4) d'un patient à un dispositif d'examen et/ou de traitement ophtalmologique (2), qui est conçue pour être fixée sous vide à l'œil (4) et/ou au dispositif d'examen et/ou de traitement (2) et qui comporte au moins une conduite de fluide (26, 28, 40) destinée à être fixée à un raccord du dispositif d'examen et/ou de traitement ophtalmologique (2), **caractérisée en ce que** la conduite de fluide (26, 28, 40) comprend un connecteur (46-50) qui est conçu comme un connecteur unidirectionnel de sorte que, après le premier démontage du connecteur unidirectionnel, aucun nouveau raccordement étanche aux fluides n'est plus possible ou **en ce que** l'interface patient (6) comporte une paroi nécessaire à la fixation sous vide et à l'intérieur un point de rupture (68) qui se brise à une pression différentielle spécifiée.

15. Interface patient destinée à relier un œil (4) d'un patient à un dispositif d'examen et/ou de traitement ophtalmologique (2), ladite interface comprenant un élément transparent (22),
- l'interface patient (6) comportant un élément transparent (22) à travers lequel le rayonnement peut être guidé vers l'œil (4) d'un patient et qui comporte une couche réactive (70) qui prend en charge une propriété optique prédéterminée lors d'une stérilisation prédéterminée ou d'une désinfection de l'interface patient (6), notamment la couche réactive (70) prenant en charge la propriété optique prédéterminée au-dessus d'une température spécifiée et/ou lors d'un contact avec un fluide désinfectant et/ou lors d'une deuxième stérilisation par rayonnement ionisant,
et/ou
- l'élément structurel comportant un matériau qui se ramollit à des températures d'autoclavage, notamment supérieures à 60, 80 ou 100 degrés Celsius.
